(19) Europäisches Patentamt
European Patent Office
Office européen des brevets

(11) EP 0 989 131 B1

(12) **EUROPEAN PATENT SPECIFICATION**

(45) Date of publication and mention of the grant of the patent:
**13.11.2002 Bulletin 2002/46**

(21) Application number: **97934774.7**

(22) Date of filing: **11.08.1997**

(51) Int Cl.[7]: **C07D 495/14**, A61K 31/55

(86) International application number:
**PCT/JP97/02817**

(87) International publication number:
**WO 98/011111 (19.03.1998 Gazette 1998/11)**

(54) **THIENOTRIAZOLODIAZEPINE COMPOUNDS AND MEDICINAL USES THEREOF**

THIENOTRIAZOLODIAZEPINVERBINDUNGEN UND IHRE MEDIZINISCHEN ANWENDUNGEN

COMPOSES DE THIENOTRIAZOLODIAZEPINE ET LEURS UTILISATIONS A DES FINS MEDICINALES

(84) Designated Contracting States:
**AT BE CH DE DK ES FI FR GB GR IE IT LI LU MC NL PT SE**
Designated Extension States:
**AL LT LV RO SI**

(30) Priority: **13.09.1996 JP 24379396**

(43) Date of publication of application:
**29.03.2000 Bulletin 2000/13**

(73) Proprietor: **Mitsubishi Pharma Corporation**
**Osaka-shi, Osaka 541-0046 (JP)**

(72) Inventors:
- **SUEOKA, Hiroyuki, Yoshitomi Pharm. Ind. Ltd.**
  **Chikujo-gun, Fukuoka 871-8550 (JP)**
- **KOMATSU, Hirotsugu,**
  **Yoshitomi Pharm. Ind. Ltd.**
  **Chikujo-gun, Fukuoka 871-8550 (JP)**
- **KOBAYASHI, Haruhito,**
  **Yoshitomi Pharm. Ind. Ltd.**
  **Chikujo-gun, Fukuoka 871-8550 (JP)**
- **EHARA, Syuji, Yoshitomi Pharm. Ind. Ltd.**
  **Chikujo-gun, Fukuoka 871-8550 (JP)**

(74) Representative:
**von Kreisler, Alek, Dipl.-Chem. et al**
**Patentanwälte,**
**von Kreisler-Selting-Werner,**
**Bahnhofsvorplatz 1 (Deichmannhaus)**
**50667 Köln (DE)**

(56) References cited:

| | |
|---|---|
| **WO-A-93/07129** | **WO-A-93/12117** |
| **WO-A-94/06801** | **WO-A-94/06802** |
| **WO-A-94/22872** | **JP-A- 2 243 691** |
| **JP-A- 2 275 883** | **JP-A- 3 215 489** |
| **JP-A- 4 261 181** | **JP-A- 7 179 471** |
| **JP-A- 62 181 282** | |

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### Technical Field

The present invention relates to thienotriazolodiazepine compounds that have strong inhibitory effect on cell adhesion and that are useful as a therapeutic drug for inflammatory bowel diseases such as ulcerative colitis, Crohn's disease and the like, and to pharmaceutical use thereof.

### Background Art

International Publication No. WO 89/05812 and Japanese Patent Unexamined Publication No. 223290/1991 disclose thienodiazepine compounds having CCK antagonistic action or gastrin antagonistic action; International Publication No. WO 93/07129 discloses a thienotriazolodiazepine compound usable as a therapeutic agent for osteoporosis; and International Publication Nos. WO 93/12177, WO94/06082 and WO 94/22872 disclose thienotriazolodiazepine compounds having inhibitory effect on cell adhesion.

Japanese Patent Unexamined Publication No. 243691/1990 discloses a thienotriazolodiazepine compound having platelet activating factor (PAF) inhibitory activity. The compounds disclosed in examples have either a substituent at the 2-position of thiophene or a cycloalkane formed by the 2-position and 3-position in combination, and phenyl having chlorine at the ortho position (2-position), i.e., 2-chlorophenyl, at the 4-position. The 6-position thereof is substituted by methyl, hydroxyl, carboxyalkyl, hydroxyalkyl, alkoxycarbonyl, alkoxycarbonylalkyl, morpholinocarbonylethyl and the like. Of these, the compound of Example 8 having the structure of the following formula (A) is known as a compound having amide at the 6-position.

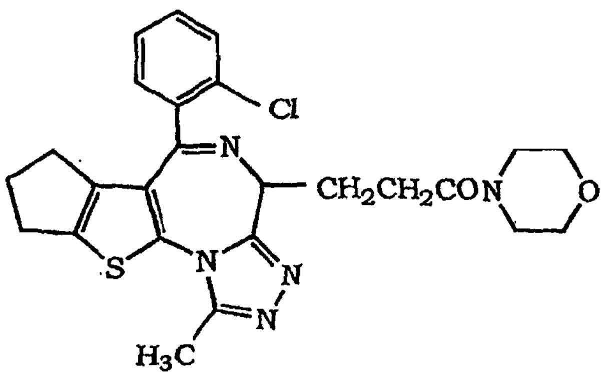

(A)

In various inflammations and allergic diseases, infiltration of so-called inflammatory cells (leukocytes in a wide sense) such as polymorphonuclear leukocytes, macrophages and lymphocytes is deeply concerned with the symptoms of the diseases. The recent progress in molecular biology has enabled identification of the molecules concerned with the adhesion, and their functional significance, that is, the phenomenon of leukocyte adhesion via certain adhesion molecules expressed on vascular endothelial cells, and adhesion molecules on leukocytes that specifically bind with said molecules, followed by infiltration of leukocytes into inflammatory cells, has been clarified.

In connection with diseases, promoted expressions of ICAM-1 (intercellular adhesion molecule-1) and E-selectin in inflammatory sites in autoimmune diseases, such as inflammatory skin diseases (e.g. contact dermatitis, light eruptions caused by high photosensitivity, and so on) and chronic rheumatoid arthritis, have been reported, and involvement of VCAM-1 (vascular cell adhesion molecule-1), ICAM-1 and Mac-1 in asthma has been suggested. It has also been reported that cell adhesion via ICAM-1 plays an important role in graft rejection after the organ transplantation, and that adhesion molecules are concerned with tumor metastasis. Recent studies have increasingly revealed that cell adhesion molecules, such as Mac-1, E-selectin, VCAM-1 and the like, are deeply concerned with the onset and progress of ulcerative colitis, Crohn's disease, nephritis and so on. Moreover, cell adhesion molecules are known to be deeply involved in the formation and evolution of atherosclerosis, ischemia-reperfusion injury, septic shock and so on.

Under these circumstances, a compound having an inhibitory action on cell adhesion is expected to be a superior antiinflammatory drug or anti-allergic drug. For example, Proc. Natl. Acad. Sci. U.S.A., vol. 88, pp 355-359 (1991) teaches that N-(fluorenyl-9-methoxycarbonyl)amino acids suppress reactions in various animal inflammatory models by inhibiting adhesion of leukocytes, and an extract from American Federation for Clinical Research Annual Meeting, May 6, 1991, teaches that the same series of compounds inhibit leukocyte adhesion to vascular endothelial

cells, by inhibiting expression of adhesion molecules (CD 18 and the like) on leukocytes. As discussed, compounds having inhibitory effect on cell adhesion are expected to be usable as pharmaceutical agents for the prevention and treatment of the above-mentioned diseases and disorders.

However, the compounds described in various known literatures including those mentioned above are not sufficient to afford sufficient inhibitory effect on cell adhesion, and the development of a compound having stronger cell adhesion inhibitory effect is desired.

Ulcerative colitis and Crohn's disease are chronic intractable diseases that have no established complete cure. For the therapy of ulcerative colitis and Crohn's disease, steroidal agents such as prednisolone, 5-aminosalicylic acid (5-ASA), Salazosulfapyridine (SASP) and the like have been used. Of these, steroidal agents often cause severe side effects such as moon face, hyperglycemia, increased urinary volume, inhibition of adrenal cortex function and the like, and other drugs provide therapeutic effects that are not necessarily sufficient. Therefore, there is a strong demand for a therapeutic agent replacing these drugs.

There have been also known antiallergic agent and antiinflammatory agent that are based on PAF antagonistic action, therapeutic agent for pancreatitis and gastric inhibitory drug based on CCK antagonistic action and gastrin antagonistic action, and therapeutic agents for inflammation, allergic disease and autoimmune disease that are based on cell adhesion inhibitory action, and the like from the aforementioned publications, but there has not been known a therapeutic agent for ulcerative colitis or Crohn's disease based on cell adhesion inhibitory action.

## Disclosure of the Invention

The present inventors have searched for a substance having a strong cell adhesion inhibitory action, with the aim of developing a therapeutic agent of inflammatory bowel diseases, such as ulcerative colitis and Crohn's disease, and found that an optically active compound of the formula (1), a pharmaceutically acceptable salt thereof or a hydrate thereof strongly inhibits the expression of Mac-1 (CD11b), VCAM-1 and E-selectin that are adhesion molecules responsible for the disease state of an inflammatory bowel disease and significantly suppresses intestinal weight increase caused by the onset of colitis in experiments using animal colitis model, which resulted in the completion of the invention.

Accordingly, the present invention provides the following.

① A thienotriazolodiazepine compound of the formula (1)

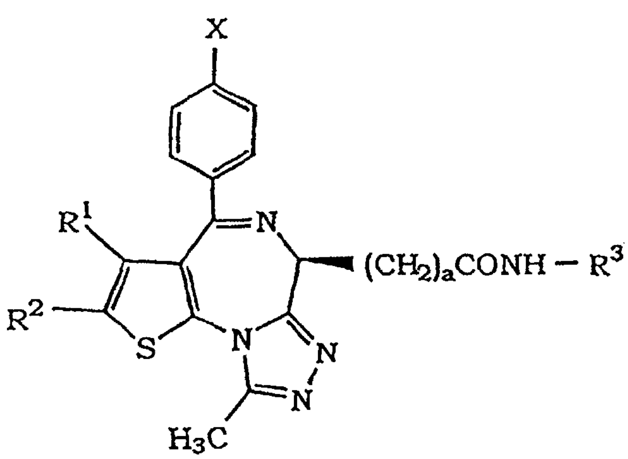

(1)

wherein

- X is a halogen;
- $R^1$ is an alkyl having 1 to 4 carbon atoms;
- $R^2$ is an alkyl having 1 to 4 carbon atoms;
- a is an integer of 1 to 4; and
- $R^3$ is an alkyl having 1 to 4 carbon atoms, a hydroxyalkyl having 1 to 4 carbon atoms, an alkoxy having 1 to 4 carbon atoms, a phenyl optionally having as a substituent 1 or 2 from halogen, alkyl having 1-4 carbon atoms, hydroxy, hydroxyalkyl having 1 to 4 carbon atoms, amino and nitro, or a heteroaryl selected from pyridyl, pyrazinyl, pyrimidinyl, thienyl and furyl, which heteroaryl optionally having as a substituent, 1 or 2 from halogen, alkyl having 1 to 4 carbon atoms, hydroxy, amino, nitro and alkoxy having 1 to 4 carbon atoms,

a pharmaceutically acceptable salt thereof or a hydrate thereof.

② The thienotriazolodiazepine compound of ① above, wherein, in the formula (1), X is a chlorine and $R^1$ and $R^2$ are both methyl, a pharmaceutically acceptable salt thereof or a hydrate thereof.

③ The thienotriazolodiazepine compound of ① or ② above, wherein, in the formula (1), a is 1, a pharmaceutically acceptable salt thereof or a hydrate thereof.

④ The thienotriazolodiazepine compound of ① above, wherein the compound of the formula (1) is (S)-2-[4-(4-chlorophenyl)-2,3,9-trimethyl-6H-thieno[3,2-f][1,2,4]triazolo[4,3-a][1,4]diazepin-6-yl]-N-(4-hydroxyphenyl)acetamide, a pharmaceutically acceptable salt thereof or a hydrate thereof.

⑤ (S)-2-[4-(4-Chlorophenyl)-2,3,9-trimethyl-6H-thieno[3,2-f][1,2,4]triazolo[4,3-a][1,4]diazepin-6-yl]-N-(4-hydroxyphenyl)acetamide dihydrate.

⑥ A medication comprising a thienotriazolodiazepine compound of the formula (1)

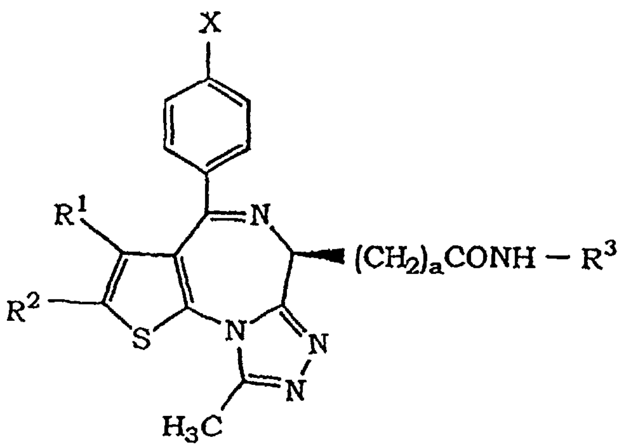

(1)

wherein

X is a halogen;
$R^1$ is an alkyl having 1 to 4 carbon atoms;
$R^2$ is an alkyl having 1 to 4 carbon atoms;
a is an integer of 1 to 4; and
$R^3$ is an alkyl having 1 to 4 carbon atoms, a hydroxyalkyl having 1 to 4 carbon atoms, an alkoxy having 1 to 4 carbon atoms, a phenyl optionally having as a substituent 1 or 2 from halogen, alkyl having 1-4 carbon atoms, hydroxy, hydroxyalkyl having 1 to 4 carbon atoms, amino and nitro, or a heteroaryl selected from pyridyl, pyrazinyl, pyrimidinyl, thienyl and furyl, which heteroaryl optionally having as a substituent, 1 or 2 from halogen, alkyl having 1 to 4 carbon atoms, hydroxy, amino, nitro and alkoxy having 1 to 4 carbon atoms,

a pharmaceutically acceptable salt thereof or a hydrate thereof.

⑦ The medication of ⑥ above, comprising a thienotriazolodiazepine compound of the formula (1), when X is a chlorine and $R^1$ and $R^2$ are both methyl, a pharmaceutically acceptable salt thereof or a hydrate thereof.

⑧ The medication of ⑥ or ⑦ above, comprising a thienotriazolodiazepine compound of the formula (1), wherein a is 1, a pharmaceutically acceptable salt thereof or a hydrate thereof.

⑨ The medication of ⑥ above, wherein the compound of the formula (1) is (S)-2-[4-(4-chlorophenyl)-2,3,9-trimethyl-6H-thieno[3,2-f][1,2,4]triazolo[4,3-a][1,4]diazepin-6-yl]-N-(4-hydroxyphenyl)acetamide.

⑩ The medication of ⑥ above, which is a therapeutic agent for an inflammatory bowel disease.

⑪ The medication of ⑥ above, which is a therapeutic agent for an ulcerative colitis.

⑫ The medication of ⑥ above, which is a therapeutic agent for Crohn's disease.

⑬ A medication comprising (S)-2-[4-(4-chlorophenyl)-2,3,9-trimethyl-6H-thieno[3,2-f][1,2,4]triazolo[4,3-a][1,4]diazepin-6-yl]-N-(4-hydroxyphenyl)acetamide dihydrate.

⑭ The medication of ⑬ above, which is a therapeutic agent for an inflammatory bowel disease.

⑮ The medication of ⑬ above, which is a therapeutic agent for an ulcerative colitis.

⑯ The medication of ⑬ above, which is a therapeutic agent for Crohn's disease.

The compound of the present invention is characterized by substituent having an amide bond which is shown

by the formula

$$—(CH_2)_aCONH—R^3$$

at the 6-position, like the formula (1) and the specific configuration (S configuration) of the substituent. Besides these, the combination of 2-, 3- and 9-positions being alkyl such as methyl, and the 4-position being 4-halogenophenyl having halogen at para position is important. The object of the present invention has been first achieved by a thienotriazolodiazepine compound having this combination of the 2-, 3-, 4-, 6- and 9-positions.

In the formula (1), each symbol means the following. Halogen at X is chlorine, bromine, fluorine or iodine, with preference given to chlorine. The alkyl having 1 to 4 carbon atoms at $R^1$, $R^2$ and $R^3$ is methyl, ethyl, propyl, isopropyl, butyl, isobutyl, sec-butyl or tert-butyl, with preference given to methyl. The hydroxyalkyl having 1 to 4 carbon atoms at $R^3$ is hydroxymethyl, 2-hydroxyethyl, 1-hydroxyethyl, 3-hydroxypropyl, 4-hydroxybutyl and the like. The alkoxy having 1 to 4 carbon atoms at $R^3$ is methoxy, ethoxy, n-propoxy, isopropoxy, n-butoxy, isobutoxy and the like. The phenyl optionally having a substituent at $R^3$ is phenyl optionally having, as a substituent, one or two from halogen (e.g., chlorine, bromine, fluorine and the like), alkyl having 1 to 4 carbon atoms (e.g., methyl, ethyl and the like), hydroxy, hydroxyalkyl having 1 to 4 carbon atoms (e.g., hydroxymethyl, hydroxyethyl and the like), amino and nitro, and is exemplified by 4-hydroxyphenyl, 4-aminophenyl, 3-chlorophenyl and the like. The heteroaryl optionally having a substituent at $R^3$ is pyridyl, pyrazinyl, pyrimidinyl, thienyl, furyl and the like, which may have, as a substituent, 1 or 2 from halogen (e.g., chlorine, bromine, fluorine and the like), alkyl having 1 to 4 carbon atoms (e.g., methyl, ethyl and the Like), hydroxy, amino, nitro and alkoxy having 1 to 4 carbon atoms (e.g., methoxy, ethoxy and the like), and is exemplified by 3-pyridyl, 2-methoxy 3-pyridyl, 4-methoxy-3-pyridyl, 1,2,3,4-tetrahydro-2-oxoquinolin-6-yl and the like.

The pharmaceutically acceptable salts of the inventive compound are, for example, acid addition salts with inorganic acid (e.g. hydrochloric acid, hydrobromic acid, sulfuric acid, phosphoric acid and nitric acid) or organic acid (e.g. acetic acid, propionic acid, succinic acid, glycolic acid, lactic acid, malic acid, tartaric acid, citric acid, maleic acid, fumaric acid, methanesulfonic acid, benzenesulfonic acid, p-toluenesulfonic acid and ascorbic acid), and salts with inorganic base (e.g., sodium hydroxide, potassium hydroxide, calcium hydroxide, magnesium hydroxide, zinc hydroxide, ammonium hydroxide and the like), organic base (e.g., methylamine, diethylamine, triethylamine, dicyclohexylamine, triethanolamine, ethylenediamine, trishydroxymethylaminomethane, quinine, guanidine, cinchonine and the like) or amino acid (e.g., lysine, ornithine, arginine, alanine and the like). In view of the object of the present invention, these salts are preferably nontoxic. In addition, hydrates (e.g., monohydrate, dihydrate and the like) and other solvates are also encompassed.

The compound of the formula (1) is preferably (S)-2-[4-(4-chlorophenyl)-2,3,9-trimethyl-6H-thieno[3,2-f][1,2,4]triazolo[4,3-a][1,4]diazepin-6-yl]-N-(4-hydroxyphenyl)acetamide of the following formula, and a dihydrate thereof is most preferable in view of stability.

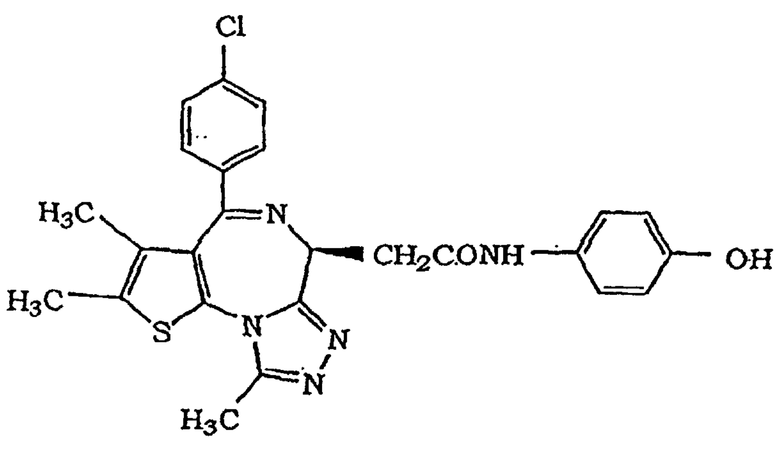

The compound of the formula (1) of the present invention can be obtained by reacting a compound of the formula (2) with a compound of the formula (3) as shown in the following reaction formulas.

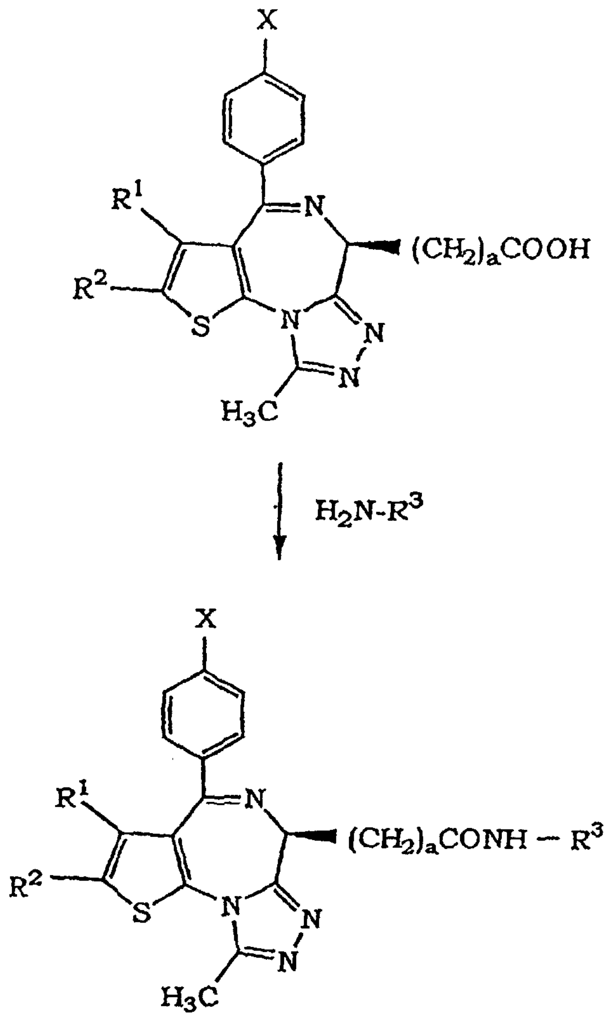


(2)

(3).

(1)

wherein each symbol is as defined above.

The reaction is carried out in a suitable solvent which does not inhibit the reaction, such as an organic solvent (e.g. tetrahydrofuran, diethyl ether, diisopropyl ether, dioxane, dichloromethane, chloroform, carbon tetrachloride, dichloroethane, ethyl acetate, benzene, toluene, xylene, dimethylformamide, dimethylacetamide and dimethylsulfoxide) in the presence of, where necessary, a base, a thionyl halide, a halide of organic acid or a condensing agent at a temperature of from -60°C to the boiling point of the solvent.

Examples of the base to be used as necessary include alkali metal hydroxides such as sodium hydroxide and potassium hydroxide, alkali metal carbonates such as sodium carbonate and potassium carbonate, alkali metal hydrogencarbonates such as sodium hydrogencarbonate and potassium hydrogencarbonate, alkali metal hydrides such as sodium hydride, alkali metal alkoxides such as sodium methoxide and potassium tert-butoxide, and organic bases such as triethylamine, pyridine, picoline and N-methylmorpholine. An alkali metal hydroxide, an alkali metal carbonate or an alkali metal hydrogencarbonate may be used in two phases of the above-mentioned organic solvent and water, using, where necessary, a phase transfer catalyst such as tetrabutylammonium bromide and benzyltriethylammonium iodide. Thionyl halide is exemplified by thionyl chloride and thionyl bromide. The halide of organic acid preferably forms a mixed acid anhydride together with the carboxylic acid of the formula (2), and is exemplified by ethyl chloroformate, isobutyl chloroformate and pivaloyl chloride. The condensing agent is preferably one used for amide synthesis, and is exemplified by dicyclohexylcarbodiimide (DCC), N-ethyl-N'-(dimethylaminopropyl)carbodiimide hydrochloride (WSC), diphenylphosphoryl azide (DPPA), N-methyl-2-chloropyridinium iodide, benzotriazol-1-yl-oxy-tris(dimethylamino)-phosphonium hexafluorophosphate (Bop reagent), 2-chloro-1,3-dimethylimidazolium chloride (DMC) and molecular sieve.

The compounds (1) of the present invention thus obtained can be separated and purified from reaction mixtures by a method known in the art, such as recrystallization and column chromatography.

The compounds of the formula (1) are treated with inorganic acid (e.g. hydrochloric acid, hydrobromic acid,

sulfuric acid, phosphoric acid and nitric acid), organic acid (e.g. acetic acid, propionic acid, succinic add, glycolic acid, lactic acid, malic acid, tartaric acid, citric acid, maleic acid, fumaric acid, methanesulfonic acid, benzenesulfonic acid, p-toluenesulfonic acid and ascorbic acid), inorganic base (e.g. sodium hydroxide, potassium hydroxide, calcium hydroxide, magnesium hydroxide, zinc hydroxide and ammonium hydroxide), organic base (e.g. methylamine, diethylamine, triethylamine, dicyclohexylamine, triethanolamine, ethylenediamine, trishydroxymethylaminomethane, quinine, guanidine and cinchonine) or amino acid (e.g., lysine, ornithine, arginine and alanine) to give salts by a conventional method.

A compound of the formula (2) can be obtained as follows. A compound of formula (4)

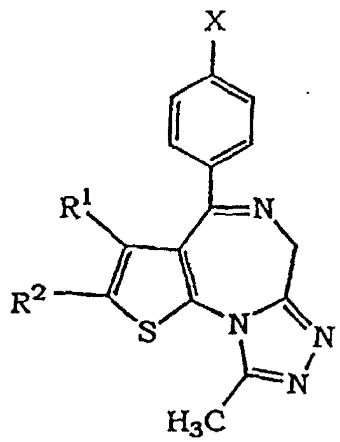

(4)

wherein each symbol is as defined above, is reacted with dialkyl carbonate such as diethyl carbonate and dimethyl carbonate in the presence of a base such as sodium ethylate, sodium methylate, sodium hydride, potassium tert-butoxide, lithium diisopropylamide and butyl lithium to introduce alkoxycarbonyl such as ethoxycarbonyl and methoxycarbonyl at the 6-position, and reacted with a haloester of the formula (S)

$$Q\text{-}(CH_2)_a\ COOR^4 \qquad (5)$$

wherein Q is a halogen such as chlorine and bromine, and $R^4$ is an alkyl such as methyl and ethyl, to give a compound of the formula (6)

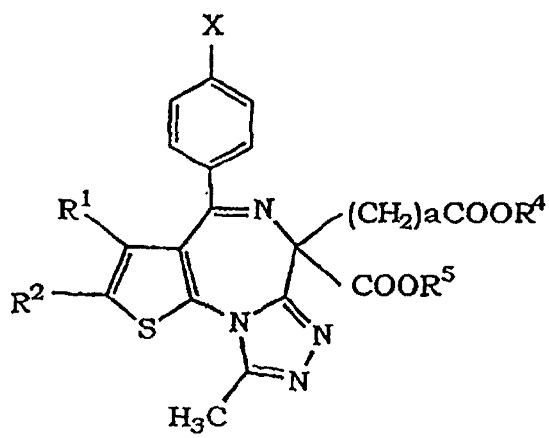

(6)

wherein $R^5$ is an alkyl such as methyl and ethyl, and other symbols are as defined above.

A compound of the formula (6) is subjected to hydrolysis in water or a mixed solvent of water and a suitable solvent (e.g. methanol, ethanol, tetrahydrofuran and dioxane) in the presence of a base such as lithium hydroxide, sodium hydroxide, potassium hydroxide, sodium carbonate, potassium carbonate, sodium hydrogencarbonate and

barium hydroxide at a temperature of from 0°C to the boiling point of the solvent used, and further to decarboxylation by making the reaction system acidic using an acid such as hydrochloric acid, sulfuric acid, acetic acid, hydrobromic acid, trifluoroacetic acid and trifluoromethanesulfonic acid, whereby a compound of the formula (7)

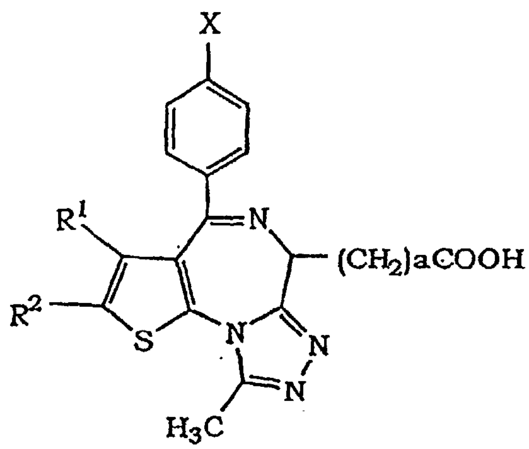

(7)

wherein each symbol is as defined above, is obtained.

From the compound of the formula (6), a compound of the formula (8)

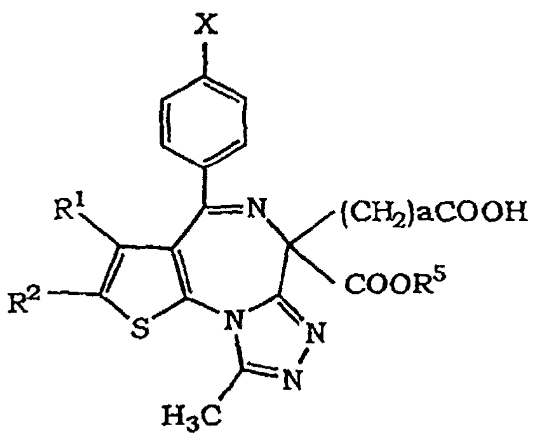

(8)

wherein each symbol is as defined above, which is an intermediate for the compound of the formula (7) can be obtained depending on the conditions of the hydrolysis.

The compound of the formula (7) can be directly obtained by reacting the compound of the formula (4) with the compound of the formula (5) in an inert solvent such as tetrahydrofuran, dioxane, diethyl ether, benzene, toluene and dimethylformamide in the presence of a base (e.g. sodium hydride, potassium tert-butoxide, lithium diisopropylamide and butyl lithium) at a temperature of from -50°C to 0°C, and subjecting the obtained compound to hydrolysis.

Optically active compounds of the formula (7) can be obtained, for example, by the following methods.

Method (A):

A compound of the formula (7) or (8) is converted to a diastereomer salt with an optically active amine, and resolved by crystallization or recrystallization.

Examples of the optically active amine to form the salt include (1R,2S)-(-)-2-amino-1,2-diphenylethanol, (1S, 2R)-(+)-2-amino-1,2-diphenylethanol, D-(-)-arginine, L-(+)-arginine, brucine, cinchonine, cinchonidine, (+)-dehydroabiethylamine, L-(+)-lysine, (R)-(+)-1-(1-naphthyl)ethylamine, (S)-(-)-1-(1-naphthyl)ethylamine, (R)-(+)-1-phenethylamine, (S)-(-)-1-phenethylamine, quinine and strychnine, and crystallization is performed in a solvent such as methanol, ethanol, 1-propanol, 2-propanol, acetone, ethyl acetate and acetonitrile. The thus-obtained optically active compound of the formula (8) is subjected to hydrolysis in water or a mixed solvent of water and a suitable solvent (e.g. methanol, ethanol, tetrahydrofuran and dioxane) in the presence of a base such as lithium hydroxide, sodium hydroxide, potassium hydroxide, sodium carbonate, potassium carbonate, sodium hydrogencarbonate and barium hydroxide at a temperature of from 0°C to the boiling point of the solvent used, and further subjected to decarboxylation using an acid such as hydrochloric acid, sulfuric acid, hydrobromic acid, citric acid, acetic acid, trifluoroacetic acid and trifluoromethanesulfonic acid, whereby an optically active compound of the formula (7) can be obtained.

Method (B):

The compound of the formula (7) or (8) is converted to a diastereomer ester with an optically active alcohol and resolved.

The compound of the formula (7) or (8) is esterified with an optically active alcohol such as (R)-(-)-2-butanol, (S)-(+)-2-butanol, (+)-menthol, (-)-menthol, (R)-(+)-1-phenylethanol and (S)-(-)-1-phenylethanol by a conventional method; resolved by column chromatography, recrystallization and the like; and subjected to hydrolysis in water or a mixed solvent of water and a suitable solvent (e.g. methanol, ethanol, tetrahydrofuran and dioxane) in the presence of a base such as lithium hydroxide, sodium hydroxide, potassium hydroxide, sodium carbonate, potassium carbonate, sodium hydrogencarbonate and barium hydroxide at a temperature of from 0°C to the boiling point of the solvent used. In the case of the compound of the formula (8), the compound is further subjected to decarboxylation using an acid such as hydrochloric acid, sulfuric acid, hydrobromic acid, citric acid, acetic acid, trifluoroacetic acid and trifluoromethanesulfonic acid, whereby an optically active compound of the formula (7) can be obtained.

Method (C):

A compound of the formula (6), (7) or (8) is converted to a diastereomer salt with an optically active sulfonic acid and resolved by crystallization or recrystallization.

Examples of the optically active sulfonic acid to form the salt include (+)-10-camphorsulfonic acid, (-)-10-camphorsulfonic acid, (+)-3-bromocamphor-8-sulfonic acid and (-)-3-bromocamphor-8-sulfonic acid. The crystallization is performed in a solvent such as methanol, ethanol, 1-propanol, 2-propanol, acetone, ethyl acetate and acetonitrile. The thus-obtained optically active compound of the formula (6) or (8) is subjected to hydrolysis in water or a mixed solvent of water and a suitable solvent (e.g. methanol, ethanol, tetrahydrofuran and dioxane) in the presence of a base such as lithium hydroxide, sodium hydroxide, potassium hydroxide, sodium carbonate, potassium carbonate, sodium hydrogencarbonate and barium hydroxide at a temperature of from 0°C to the boiling point of the solvent used, and further subjected to decarboxylation using an acid such as hydrochloric acid, sulfuric acid, hydrobromic acid, citric acid, acetic acid, trifluoroacetic add and trifluoromethanesulfonic acid, whereby an optically active compound of the formula (7) can be obtained.

The above-mentioned compound of the formula (4) is synthesized as in the following.

A compound of the formula (10)

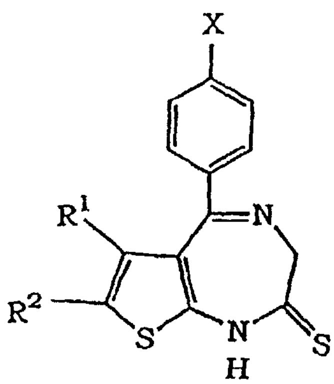

(10)

wherein each symbol is as defined above, which is obtained using a compound of the formula (9)

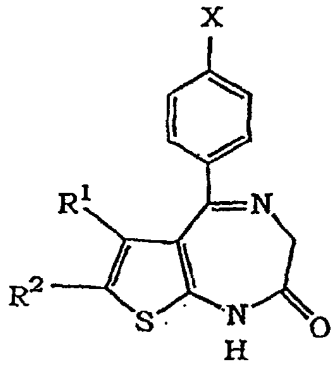

(9)

wherein each symbol is as defined above, by the method described in Japanese Patent Unexamined Publication Nos. 79185/1989 and 156982/1989, and a compound of the formula (11)

$$H_3CCONHNH_2 \quad (11)$$

are reacted, whereby a compound of the formula (4) is produced.

Alternatively, a compound of the formula (12)

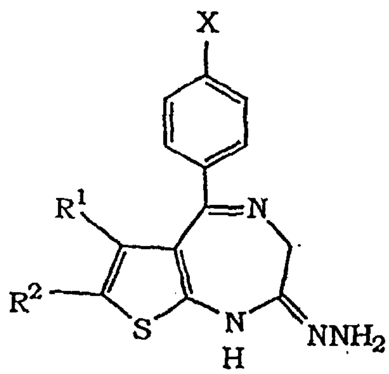

(12)

wherein each symbol is as defined above, which is obtained by reacting a compound of the formula (10) with a hydrazine hydrate, is reacted with acetic acid of the formula (13)

$$H_3CCOOH \quad (13)$$

a reactive derivative thereof or a compound of the formula (14)

$$H_3CC(OR^6)_3 \quad (14)$$

wherein $R^6$ is an alkyl such as methyl and ethyl, whereby a compound of the formula (4) is produced.

The reaction between the compound of the formula (10) and the compound of the formula (11) generally proceeds in a solvent inert to the reaction (e.g. benzene, toluene, xylene, tetrahydrofuran, dioxane and a mixed solvent thereof), in the presence of an organic acid (e.g. acetic acid and propionic acid), an inorganic acid (e.g. hydrochloric acid and sulfuric acid) or silica gel at a temperature of from room temperature to the refluxing temperature of the solvent used, for 30 minutes to 5 hours. The reaction between the compound of the formula (10) and the hydrazine hydrate generally proceeds in a solvent inert to the reaction (e.g. methanol, ethanol, propanol, isopropyl alcohol and butanol) at 0-40°C for 5 minutes to 3 hours.

The reaction between the compound of the formula (12), and the compound of the formula (13), its reactive

derivative or the compound of the formula (14) proceeds in a solvent inert to the reaction (e.g. benzene, toluene, xylene, tetrahydrofuran, dioxane and a mixed solvent thereof), in the presence of an organic acid (e.g. acetic acid and propionic acid), an inorganic acid (e.g. hydrochloric acid and sulfuric acid) or silica gel at a temperature from room temperature to the refluxing temperature of the solvent used, for 30 minutes to 6 hours using a Dean-Stark trap where the case demands.

The compound of the formula (1) of the present invention obtained as above and a pharmaceutical agent containing the same are useful as a therapeutic agent, a preventive drug or a recurrence preventive drug for inflammatory bowel diseases caused by cell adhesion, such as ulcerative colitis and Crohn's disease, in mammals inclusive of human, monkey, cow, horse, dog, cat, rabbit, rat and the like. The inventive compound can be used for the prophylaxis and treatment of venous insufficiency and venous ulcer or maintained remission of the both.

When the inventive compound is used as a pharmaceutical agent, the inventive compound is admixed with pharmaceutically acceptable carriers (e.g., excipients, binders, disintegrators, correctives, corrigents, emulsifying agents, diluents and solubilizers) to give a pharmaceutical composition or a pharmaceutical preparation (e.g., tablets, pills, capsules, granules, powders, syrups, emulsions, elixirs, suspensions, solutions, injections, transfusions and suppositories) which can be administered orally or parenterally to patients.

The pharmaceutical composition can be formualted into a pharmaceutical preparation by a conventional method. In the present specification, by parenteral is meant subcutaneous injection, intravenous *injection, intramuscular* injection, intraperitoneal injection, transfusion and the like. A preparation for injection such as sterile aqueous and oily suspensions for injection can be prepared by a method known in this field using a suitable dispersing agent, wetting agent and suspending agent. The sterile preparation for injection may be a sterile injectable solution (e.g., aqueous solution) or suspension in a diluent or solvent that is nontoxic and parenterally administerable. Examples of usable vehicle and solvent include water, Ringer solution, isotonic brine and the like. In addition, sterile nonvolatile oil can be generally used as a solvent or a suspending solvent. Any nonvolatile oil or fatty acid can be used for this end, and examples thereof include natural, synthetic or semisynthetic lipid oil or fatty acid, and natural, synthetic or semisynthetic mono-, di- or triglycerides.

A suppository for rectal administration can be prepared upon mixing a drug with a suitable nonirritant vehicle, such as cocoa butter and polyethylene glycols, which are solid at normal temperature and liquid at a temperature of the intestine and which melt in rectum to release the drug.

The solid dosage form for oral administration may be, for example, powder, granule, tablet, pill or capsule mentioned above. In these dosage forms, the active compound can be admixed with at least one additive, such as sucrose, lactose, cellulose sugar, mannitol, maltitol, dextran, starches, agar, alginates, chitins, chitosans, pectins, tragacanth gum, gum arabic, gelatins, collagens, casein, albumin, synthetic or semisynthetic polymers and glycerols. A product having such a dosage form may further contain different additives as usual, such as.inert diluents, lubricants such as magnesium stearate, preservatives such as sodium p-hydroxybenzoate and sorbic acids, antioxidants such as ascorbic acid, $\alpha$-tocopherol and cysteine, disintegrators, binders, thickeners, buffers, sweeteners, flavors, perfumes and the like. Tablets and pills may be further enteric coated.

The liquid preparation for oral administration is exemplified by pharmaceutically acceptable emulsion, syrup, elixir, suspension, solution and the like, which may contain an inert diluent normally used in this field, such as water.

The dose is determined in consideration of age, body weight, general condition of health, sex, diet, administration time, administration route, excretion rate, combination of drugs, disease state being treated at that time of the patient and other factors. The inventive compound, a pharmaceutically acceptable salt thereof and a hydrate thereof are low toxic and can be used safely. The daily dose, which is subject to change according to the condition and body weight of patient, the kind of compound, administration route and the like, is 0.1-500 mg/patient/day for subcutaneous, intravenous, intramuscular or intrarectal administration, and 0.5-1000 mg/patient/day for oral administration.

The present invention is described in more detail in the following by referring to Starting Material Preparation Examples, Examples, Formulation Examples and Experimental Examples, to which the present invention is not limited.

**Starting Material Preparation Example 1**

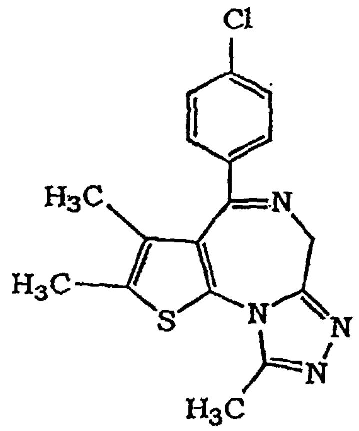

4-Chlorophenyl cyanomethyl ketone, morpholine and ethyl methyl ketone were dissolved in ethanol, and sulfur was suspended. The suspension was refluxed under heating for 10 hours. After the completion of the reaction, the solvent was evaporated under reduced pressure, and the residue was dissolved in chloroform. The mixture was washed with water, and dried over anhydrous magnesium sulfate. Chloroacetyl chloride was dropwise added, and the mixture was refluxed under heating for 1 hour. After the completion of the reaction, the resulting mixture was washed with a saturated aqueous solution of sodium hydrogencarbonate and dried over anhydrous magnesium sulfate, and the solvent was evaporated under reduced pressure. Methanol was added to the residue and crystallized to give N-(2-(3-(4-chlorobenzoyl)-4,5-dimethyl)thienyl)chloroacetamide.
This compound was dissolved in tetrahydrofuran, and sodium iodide was suspended therein. The suspension was refluxed under heating for 2 hours.
The reaction mixture was cooled to -50°C with dry ice-acetone, and liquid ammonia was added, followed by stirring. After the completion of the reaction, the solvent was evaporated under reduced pressure, and the residue was dissolved in ethyl acetate. After washing with water, the mixture was dried over anhydrous magnesium sulfate, and the solvent was evaporated under reduced pressure.
The residue was dissolved in isopropyl alcohol, and acetic acid was added. The mixture was refluxed under heating for 5 hours. The solvent was evaporated under reduced pressure, and the residue was dissolved in chloroform. The mixture was washed with a saturated aqueous solution of sodium hydrogencarbonate, and dried over anhydrous magnesium sulfate. The solvent was evaporated under reduced pressure, and crystallization from ethyl acetate afforded 5-(4-chlorophenyl)-6,7-dimethyl-1,2-dihydro-3H-thieno[2,3-e][1,4]diazepin-2-one.

5-(4-Chlorophenyl)-6,7-dimethyl-1,2-dihydro-3H-thieno[2,3-e][1,4]diazepin-2-one was dissolved in chloroform, and diphosphorus pentasulfide was added with stirring. The mixture was refluxed under heating for 3 hours. After the completion of the reaction, the reaction mixture was neutralized with a saturated aqueous solution of sodium hydrogencarbonate, washed with water and dried over anhydrous magnesium sulfate. The solvent was evaporated under reduced pressure, and the residue.was suspended in methanol. To the suspension was added 100% hydrazine hydrate under cooling, and the mixture was stirred at room temperature for 2 hours. After the completion of the reaction, precipitated crystals were collected by filtration to give 5-(4-chlorophenyl)-6,7-dimethyl-1,2-dihydro-3H-thieno[2,3-e][1,4] diezepin-2-hydrazone, melting point 226°C (decomposition). This compound was suspended in toluene, and triethyl orthoacetate was added. The mixture was stirred at 80°C for 4 hours. After the completion of the reaction, the solvent was evaporated under reduced pressure, and obtained crude crystals were recrystallized from ethyl acetate to give 4-(4-chlorophenyl)-2,3,9-trimethyl-6H-thieno[3,2-f][1,2,4]triazolo[4,3-a][1,4]diazepine melting point not less than 250°C.
$^{1}$H-NMR ($CDCl_3$, ppm) δ: 1.64 (s,3H), 2.39 (s,3H), 2.63 (s,3H), 4.06 (d,1H,J=12Hz), 5.06 (d,1H,J=12Hz), 7.22-7.44 (m,4H)

**Starting Material Preparation Example 2**

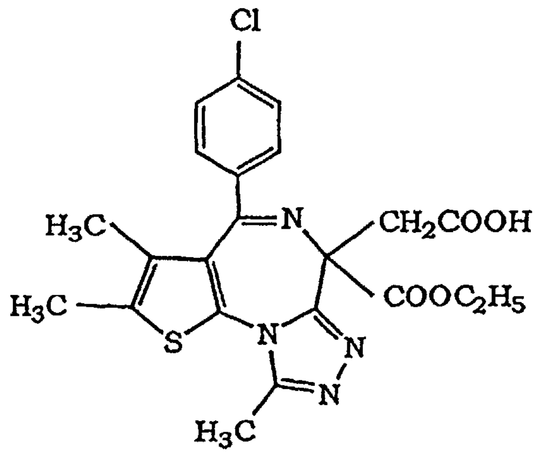

4-(4-Chlorophenyl)-2,3,9-trimethyl-6H-thieno[3,2-f][1,2,4]triazolo[4,3-a][1,4]diazepine (50 g) was dissolved in diethyl carbonate (500 ml) in a nitrogen stream, and 60% sodium hydride was added at room temperature with stirring. After refluxing under heating for 2 hours, the reaction mixture was cooled with ice water, and ethyl bromoacetate (20 ml) was added. After stirring at room temperature for 4 hours, the reaction mixture was poured into a cool 5% aqueous solution of acetic acid, and extracted with chloroform. After washing with water, the extract was dried over anhydrous magnesium sulfate, and filtered. The filtrate was concentrated under reduced pressure, and the residue was subjected to silica gel column chromatography. The objective fraction was concentrated under reduced pressure, and obtained crystals (95 g) were suspended in ethanol (910 ml). The suspension was stirred at 5°C under ice-cooling, and a 1N aqueous solution of sodium hydroxide (570 ml) was added.

After stirring at room temperature for 7 hours, the resulting mixture was neutralized with acetic acid (90 ml), and concentrated under reduced pressure.

The obtained crude crystals were recrystallized from a mixed solvent of ethanol (400 ml) - water (250 ml) to give 71 g of (±)-[4-(4-chlorophenyl)-6-ethoxycarbonyl-2,3,9-trimethyl-6H-thieno[3,2-f][1,2,4]triazolo[4,3-a][1,4]diazepin-6-yl]acetic acid, melting point 198-202°C.

**Starting Material Preparation Example 3**

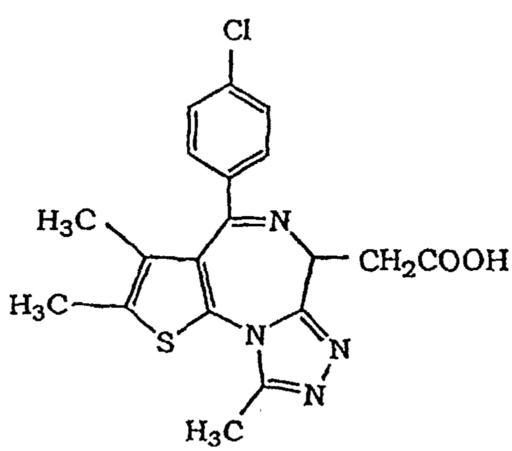

Ethanol (500 ml) was added to (±)-[4-(4-chlorophenyl)-6-ethoxycarbonyl-2,3,9-trimethyl-6H-thieno[3,2-f][1,2,4]triazolo[4,3-a][1,4]diazepin-6-yl]acetic acid (43 g), and the mixture was stirred at room temperature. 2N Sodium

hydroxide (182 ml) was added and the mixture was stirred at 50°C overnight. After the completion of the reaction, ethanol was evaporated, and the residue was adjusted to pH 4-5 with acetic acid. Then, the resulting mixture was stirred at 60°C for 30 minutes and cooled to room temperature. The mixture was extracted with chloroform, and the organic layer was washed with saturated brine. The layer was dried over anhydrous magnesium sulfate and concentrated under reduced pressure. The obtained crystals were recrystallized from chloroform-methanol to give 20 g of (±) -[4-(4-chlorophenyl)-2,3,9-trimethyl-6H-thieno[3,2-f][1,2,4]triazolo[4,3-a][1,4]diazepin-6-yl]acetic acid, melting point 287-290°C.

**Starting Material Preparation Example 4**

S-isomer

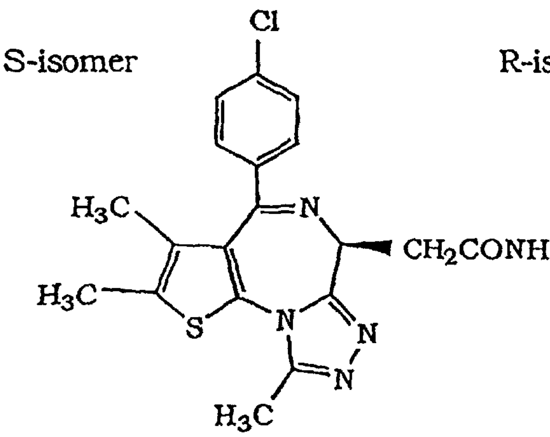

R-isomer

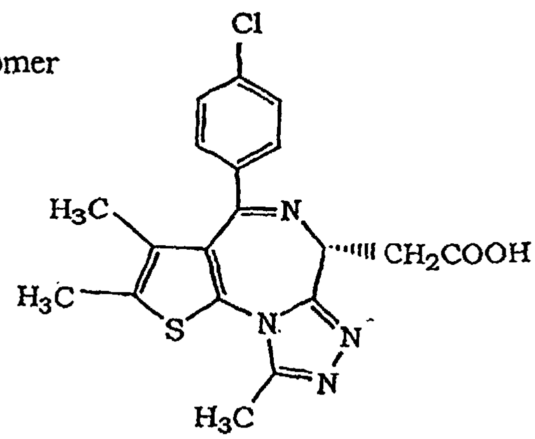

(±)-(4-(4-Chlorophenyl)-6-ethoxycarbonyl-2,3,9-trimethyl-6H-thieno[3,2-f][1,2,4]triazolo[4,3-a][1,4)diazepin-6-yl]acetic acid (82 g) and cinchonidine (50 g) were thoroughly dissolved in methanol (11), and the solvent was evaporated to give an amorphous product. Ethyl acetate (980 ml) was added and the amorphous product was dissolved in a similar way. The mixture was left standing overnight at room temperature, and a salt precipitated was removed by filtering by suction. The solvent was evaporated from the filtrate (76% ee) under reduced pressure, and chloroform (500 ml) was added to the residue. The mixture was washed twice with 10% hydrochloric acid, and the organic layer was dried over anhydrous magnesium sulfate and concentrated under reduced pressure. Ethyl acetate (3720 ml) was added to the residue (62 g), and the residue was completely dissolved. The resulting mixture was left standing overnight at room temperature, and a precipitated racemate was removed by filtering by suction. The solvent was evaporated under reduced pressure from the filtrate, and ethanol (500 ml) was added to the residue (43 g) (94% ee). A 2N aqueous solution (182 ml) of sodium hydroxide was added at room temperature with stirring, and the mixture was stirred at 50°C overnight. After the completion of the reaction, ethanol was evaporated, and the resulting mixture was adjusted to pH 4 with acetic acid. The mixture was stirred at 60°C for 30 minutes. The reaction mixture was extracted with chloroform, and the organic layer was washed with saturated brine and dried over anhydrous magnesium sulfate. After the layer was concentrated under reduced pressure, ethanol (1920 ml) was added to the residue (32 g) (92% ee) and the mixture was stirred. The insoluble racemate was removed by filtering by suction, and the solvent was evaporated under reduced pressure from the filtrate. The residue was crystallized from chloroform to give an S-isomer [(S)-[4-(4-chlorophenyl)-2,3,9-trimethyl-6H-thieno[3,2-f][1,2,4]triazolo[4,3-a][1,4]diazepin-6-yl]acetic acid] (20 g), melting point 167-169°C, as white powdery crystals (>99% ee). An R-isomer [(R)-[4-(4-chlorophenyl)-2,3,9-trimethyl-6H-thieno[3,2-f][1,2,4]triazolo[4,3-a][1,4]diazepin-6-yl]acetic acid], melting point 166-170°C, was obtained by similarly treating the crystals precipitated as a cinchonidine salt.

Optical purity (ee) was determined by HLPC under the following conditions:

Determination conditions:

column used ··· Ultron ES-OVM (analytical column)
mobile phase ···
1/15 aqueous potassium dihydrogenphosphate solution :
1/15 aqueous disodium hydrogenphosphate solution :

acetonitrile = 333:500:120
flow rate ··· 1 ml/minute
Retention time ··· about 4.5 minutes (S-isomer)
about 5.5 minutes (R-isomer)

**Example 1**

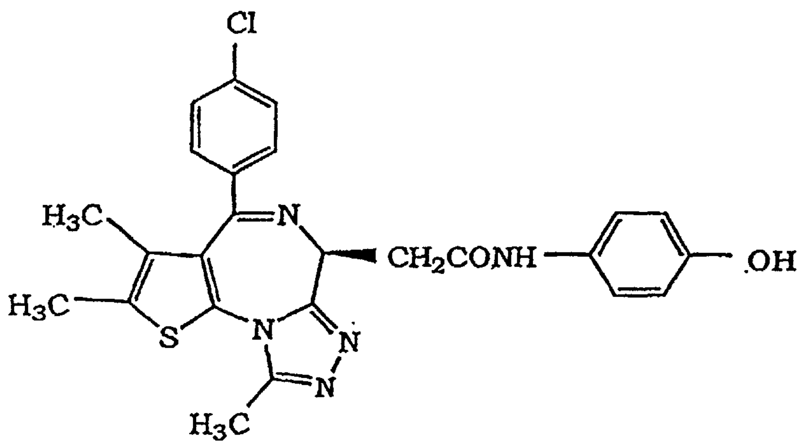

(S)-[4-(4-Chlorophenyl)-2,3,9-trimethyl-6H-thieno[3,2-f][1,2,4]triazolo[4,3-a][1,4]diazepin-6-yl]acetic acid (3.0 g) and triethylamine (1.2 ml) were added to a mixed solvent of dimethylformamide (30 ml) and tetrahydrofuran (15 ml). The mixture was cooled to -10°C, and pivaloyl chloride (1.0 ml) was dropwise added at said temperature. Thirty minutes later, a solution of 4-aminophenol (0.99 g) in dimethylformamide (20 ml) was dropwise added at said temperature, and the mixture was stirred at room temperature for 7.5 hours. After the completion of the reaction, tetrahydrofuran was evaporated, and saturated brine (200 ml) was added. The mixture was extracted with ethyl acetate, and the organic layer was washed with saturated brine, dried over anhydrous magnesium sulfate and concentrated under reduced pressure. The residue was purified by silica gel column chromatography (ethyl acetate: methanol = 10 : 1) and crystallized from isopropyl alcohol to give 620 mg of (S)-2-[4-(4-chlorophenyl)-2,3,9-trimethyl-6H-thieno[3,2-f][1,2,4]triazolo[4,3-a][1,4]diazepin-6-yl]-N-(4-hydroxyphenyl)acetamide as pale-pink powdery crystals, melting point 215-220°C.

**Example 2**

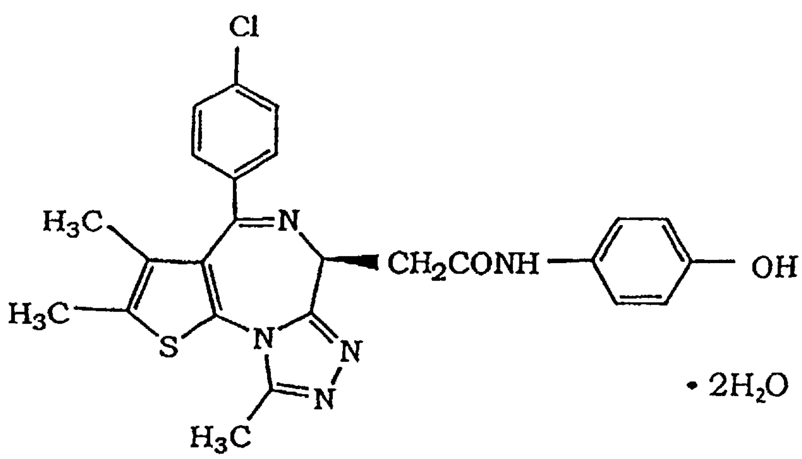

(S)-2-[4-(4-Chlorophenyl)-2,3,9-trimethyl-6H-thieno[3,2-f][1,2,4]triazolo[4,3-a][1,4]diazepin-6-yl]-N-(4-hydroxyphenyl)acetamide (5 g) was dissolved in ethanol (25 ml), and water (25 ml) was added by portions while heating at 50°C. The precipitated crystals were collected by filtration to give 4.8 g of (S)-2-[4-(4-chlorophenyl)-2,3,9-trimethyl-6H-thieno[3,2-f][1,2,4]triazolo[4,3-a][1,4]diazepin-6-yl]-N-(4-hydroxyphenyl)acetamide dihydrate, melting point 231.4°C.
$[\alpha]_D^{25}$=-19.2° (1%, methanol)

**Example 3**

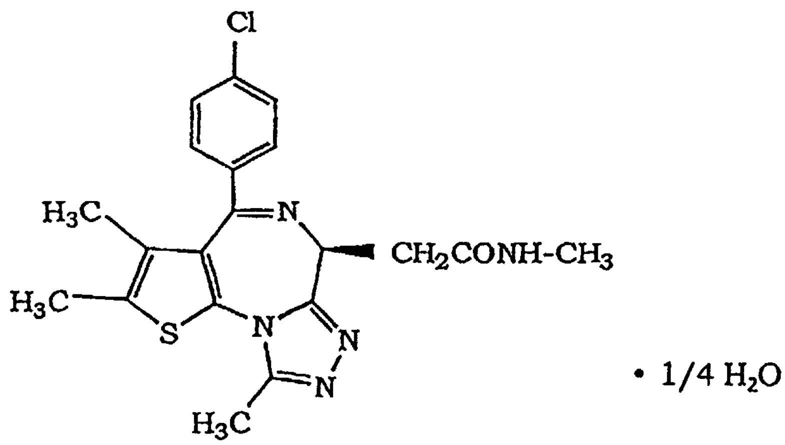

(S)-[4-(4-Chlorophenyl)-2,3,9-trimethyl-6H-thieno[3,2-f][1,2,4]triazolo[4,3-a][1,4]diazepin-6-yl]acetic acid (7.0 g) and triethylamine (2.73 ml) were added to a mixed solvent of dimethylformamide (70 ml) and tetrahydrofuran (30 ml). The mixture was cooled to -5°C, and pivaloyl chloride (2.38 ml) was dropwise added at said temperature. Ten minutes later, a solution of methylamine (0.69 g) in dimethylformamide (5 ml) was dropwise added at said temperature, and the mixture was stirred at room temperature for 2 hours. Water was added to the reaction mixture, and the mixture was extracted with ethyl acetate. The extract was washed with water and dried over anhydrous magnesium sulfate. The solvent was evaporated, and the residue was recrystallized from water/methanol to give 2.29 g of (S)-2-[4-(4-chlorophenyl)-2,3,9-trimethyl-6H-thieno[3,2-f][1,2,4]triazolo[4,3-a][1,4]diazepin-6-yl]-N-methylacetamide 1/4 hydrate, melting point 143-145°C.
$[\alpha]_D^{26}$=+18.4° (c=1, methanol)

**Example 4**

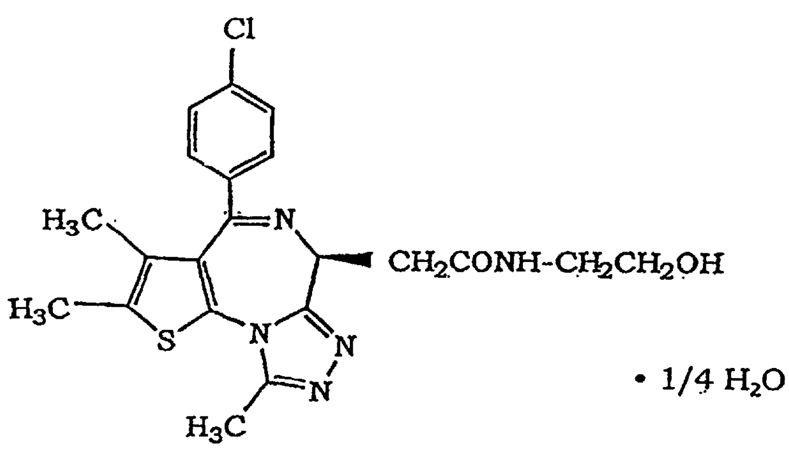

(S)-[4-(4-Chlorophenyl)-2,3,9-trimethyl-6H-thieno[3,2-f][1,2,4]triazolo[4,3-a][1,4]diazepin-6-yl]acetic acid

(7.0 g) and triethylamine (2.73 ml) were added to a mixed solvent of dimethylformamide (70 ml) and tetrahydrofuran (30 ml). The mixture was cooled to -5°C, and pivaloyl chloride (2.38 ml) was dropwise added at said temperature. Ten minutes later, a solution of ethanolamine (1.17 g) in tetrahydrofuran (10 ml) was dropwise added at said temperature, and the mixture was stirred at room temperature for 2 hours. Water was added to the reaction mixture, and the mixture was extracted with ethyl acetate. The extract was washed with water and dried over anhydrous magnesium sulfate. The solvent was evaporated, and the residue was purified by column chromatography (chloroform: methanol = 50 : 1) and recrystallized from ethyl acetate to give 2.29 g of (S)-2-[4-(4-chlorophenyl)-2,3,9-trimethyl-6H-thieno[3,2-f][1,2,4]triazolo[4,3-a][1,4]diazepin-6-yl]-N-(2-hydroxyethyl)acetamide 1/4 hydrate, melting point 254-255°C.
$[\alpha]_D^{26}$=+24.9° (c=1, methanol)

**Example 5**

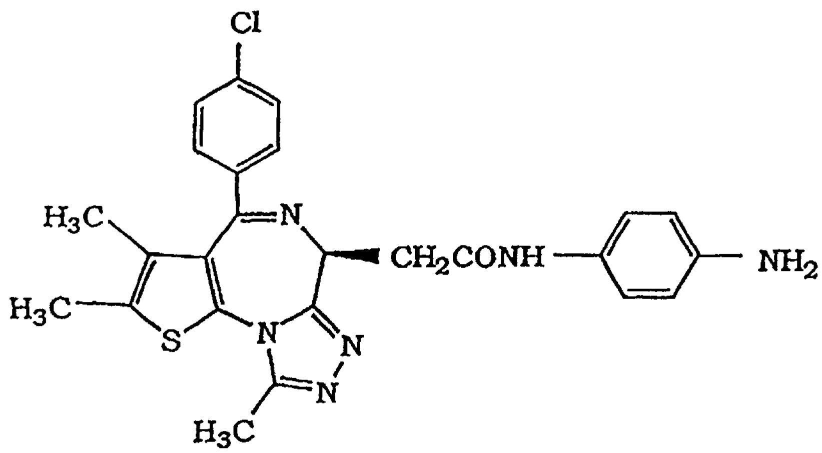

(S)-[4-(4-Chlorophenyl)-2,3,9-trimethyl-6H-thieno[3,2-f][1,2,4]triazolo[4,3-a][1,4]diazepin-6-yl]acetic acid (3.0 g), triethylamine (1.56 ml) and p-phenylenediamine (0.97 g) were dissolved in dimethylformamide (30 ml). Benzotriazol-1-yl-oxy-tris(dimethylamino)phosphonium hexafluorophosphate (3.6 g) was added, and the mixture was stirred at room temperature for 12 hours. After the completion of the reaction, water (200 ml) was added, and the mixture was extracted with ethyl acetate. The extract was washed with saturated brine and dried over anhydrous magnesium sulfate. After the solvent was evaporated under reduced pressure, the residue was subjected to silica gel column chromatography and obtained crystals were recrystallized from toluene to give 0.56 g of (S)-N-(4-aminophenyl)-2-[4-(4-chlorophenyl)-2,3,9-trimethyl-6H-thieno-[3,2-f][1,2,4]triazolo[4,3-a][1,4]diazepin-6-yl]acetamide, melting point 169-173°C.

**Example 6**

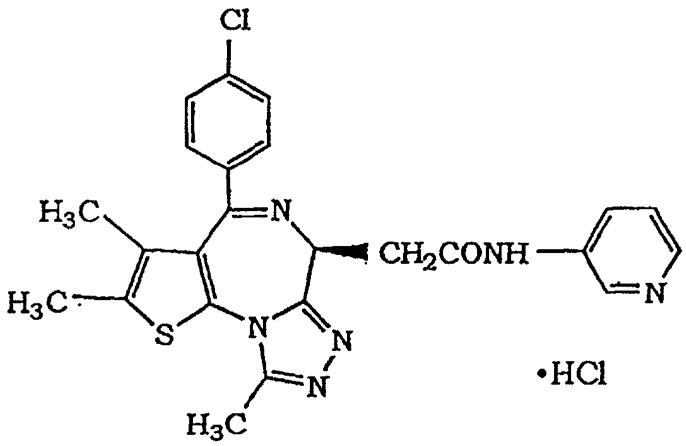

(S)-[4-(4-Chlorophenyl)-2,3,9-trimethyl-6H-thieno[3,2-f][1,2,4]triazolo[4,3-a][1,4]diazepin-6-yl]acetic acid (2.64 g) was dissolved in dimethylformamide (30 ml). The mixture was cooled in an ice bath and 1-hydroxybenzotriazole (1.08 g), N-dimethylaminopropyl-N'-ethylcarbodiimide hydrochloride (1.51 g) and triethylamine (1.08 ml) were added successively, which was followed by stirring. Five minutes later, 3-aminopyridine (0.66 g) was added to the reaction mixture and the mixture was stirred at room temperature overnight. The reaction mixture was poured into water, and the obtained crystals were purified by column chromatography (chloroform : methanol 50 : 1). The crystals were converted to hydrochloride with ethanol/hydrochloric acid in ethyl acetate, and recrystallized from ethanol/ethyl acetate to give 0.88 g of (S)-2-[4-(4-chlorophenyl)-2,3,9-trimethyl-6H-thieno[3,2-f][1,2,4]triazolo[4,3-a][1,4]diazepin-6-yl]-N-(3-pyridyl)acetamide hydrochloride, melting point 198-201°C.
$[\alpha]_D^{25}$=-22.6° (c=1, methanol)
The following compounds were produced in the same manner as in the above Examples.

**Example 7**

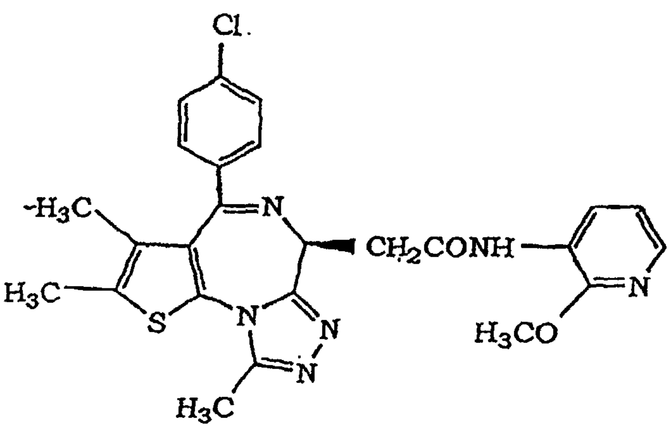

(S)-2-[4-(4-Chlorophenyl)-2,3,9-trimethyl-6H-thieno[3,2-f][1,2,4]triazolo[4,3-a][1,4]diazepin-6-yl]-N-(2-methoxy-3-pyridyl)acetamide
$^1$H-NMR 270MHz($CDCl_3$)δ: 1.69 (s,3H), 2.41 (s,3H), 2.68 (s,3H), 3.66 (m,2H), 4.00 (s,1H), 4.65 (t,H), 6.88 (dd,1H), 7.39 (dd,4H), 7.85 (dd,1H), 8.60 (dd,1H), 8.67 (br.s,1H)

**Example 8**

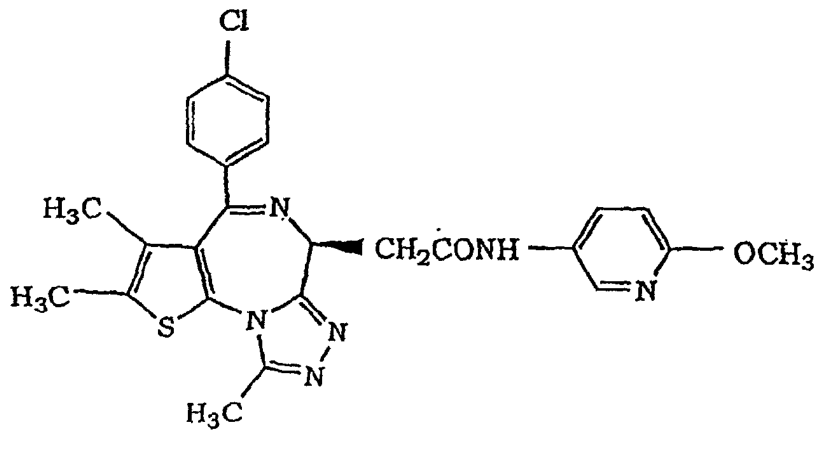

(S)-2-[4-(4-Chlorophenyl)-2,3,9-trimethyl-6H-thieno[3,2-f][1,2,4]triazolo[4,3-a][1,4]diazepine-6-yl]-N-(4-methoxy-3-pyridyl)acetamide, melting point 281-283°C

**Example 9**

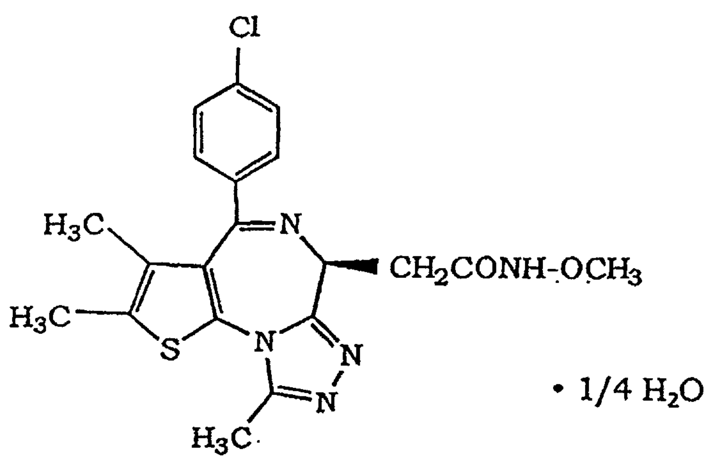

(S)-2-[4-(4-Chlorophenyl)-2,3,9-trimethyl-6H-thieno[3,2-f][1,2,4]triazolo[4,3-a][1,4]diazepin-6-yl]-N-methoxyacetamide 1/4 hydrate, melting point 230-231°C
$[\alpha]_D^{26}$=+13.7° (c=1.0, methanol)

**Formulation Example 1**

The inventive compound (0.5 part); lactose (25 parts), crystalline cellulose (35 parts) and corn starch (3 parts) were thoroughly admixed, and kneaded well with a binder prepared from corn starch (2 parts). The kneaded product was passed through a 16-mesh sieve, dried in an oven at 50°C and passed through a 24-mesh sieve. The kneaded product thus obtained, corn starch (8 parts), crystalline cellulose (11 parts) and talc (9 parts) were kneaded well, compressed into tablets containing 0.5 mg of the active ingredient per tablet The obtained tablets may be applied with enteric coating to give enteric coated tablets, which may be further prepared into sugar-coated tablets.

**Formulation Example 2**

The inventive compound (1 part) and lactose (90 parts) were thoroughly admixed, and kneaded well with a binder prepared from a suitable amount of methylcellulose. The kneaded product is passed through a 16-mesh sieve and dried in an oven at 50°C. The dried granules were forced to.pass through a 32-mesh sieve, and admixed well with an appropriate amount of silicon dioxide to give a 1% powder.

The pharmacological action of the compound of the present invention was shown in the following. In the experiments,(S)-2-[4-(4-chlorophenyl)-2,3,9-trimethyl-6H-thieno[3,2-f][1,2,4]triazolo[4,3-a][1,4]diazepin-6-yl]-N-(4-hydroxyphenyl)acetamide (hereinafter to be referred to as Compound 1) and (S)-2-[4-(4-chlorophenyl)-2,3,9-trimethyl-6H-thieno[3,2-f][1,2,4]triazolo[4,3-a][1,4]diazepin-6-yl]-N-(4-hydroxyphenyl)acetamide dihydrate (hereinafter to be referred to as Compound 2) were used as test compounds. As a control compound in Experiment 1 and Experiment 2, the compound of the formula (A) of Example 8 of Japanese Patent Unexamined Publication No. 243691/1990, i.e., [5-(2-chlorophenyl)-7-(morpholinocarbonylethyl)-10-methyl-3,4-dihydro-2H,7H-cyclopenta[4,5]thieno[3,2-f][1,2,4]triazolo[4,3-a][1,4]diazepine, hereinafter to be referred to as Comparison Compound] was used.

**Experiment 1:** Effect on CD11b expression of human histiocytic leukemia cell line U937

Effect on the expression of CD11b antigen, which constitutes the α chain of Mac-1, which is one of the adhesion molecules expressing on the surface of leukocytic cells, was investigated. U937 cells were suspended in RPMI1640 medium containing 20% fetal calf serum, plated with phorbol 12,13-dibutyrate (PDB, 10 ng/ml) and the test compound into 96-well filtration plates (30000 cells/well), and cultured for 3 days at 37°C in 5% $CO_2$. After washing the cells once with RPMI1640 medium, rat anti-human CD11b monoclonal antibody (2 μg/ml) was added and the plates were stood for 1 hr under ice-cooling. The cells were washed twice and peroxidase-conjugated anti-rat immunoglobulin antibody (1 μg/ml) was added. The cells were stood for 1 hr under ice-cooling. The cells were washed 3 times and peroxidase substrate (o-phenylenediamine) for coloring was added. The plates were stood for 15 min at room temperature and absorbance at 490 nm was measured with a 96-well microplate reader, which was used as an indicator of CD 11b antigen expression.

Table 1 shows $IC_{50}$ values (μM) obtained by calculating % inhibition when the absorbance with or without the addition of PDB was taken as 100% and 0%, respectively.

Table 1

| Compound | Inhibitory effect on CD11b expression ($IC_{50}$, μM) |
|---|---|
| Compound 2 | <0.03 |
| Comparison Compound | >3 |

As shown in Table 1, Compound 2 was clarified to inhibit CD 11b expression in U937 cells. On the other hand, the Comparison Compound showed the activity of not more than 1/100 of the activity of Compound 2.

**Experiment 2:** Inhibitory effect on expression of VCAM-1 and E-selectin in human umbilical vein-derived endothelial cells (HUVEC)

HUVEC was suspended in 199 medium containing 20% fetal calf serum, 20 μg/ml endothelial cell growth factor derived from bovine brain, and 100 μg/ml heparin, plated into 96-well microculture plates coated with collagen, and cultured at 37°C in 5% $CO_2$. When cells grew to confluence, the cells received tumour necrosis factor α (20 U/ml for VCAM-1 expression induction, 100 U/ml for E-selectin expression induction) and the test compound, which was followed by further culture for 5 hr. The cells were washed once with 199 medium and mouse anti-human VCAM-1 monoclonal antibody (2 μg/ml) or rat anti-human E-selectin monocolnal antibody (2 μg/ml) was added. The cells were stood for 1 hr at room temperature. The cells were washed twice and peroxidase-conjugated anti-mouse immunoglobulin antibody or anti-rat immunoglobulin antibody was added. The cells were stood for 1 hr at room temperature. The cells were washed 3 times and 2,2'-azino-bis(3-ethylbenzothiazoline-6-sulfonic acid) as a peroxidase substrate was added. The plates were stood for 15 min at room temperature and absorbance at 405 n/490 nm was measured with a 96-well microplate reader, which was used as an indicator of VCAM-1 or E-selectin expression.

Table 2 shows $IC_{50}$ values (μM) obtained by calculating % inhibition when the absorbance with or without the addition of tumor necrosis factor α was taken as 100% and 0%, respectively.

Table 2

| Compound | Inhibitory effect ($IC_{50}$, μM) on | |
|---|---|---|
| | VCAM-1 expression | E-selectin expression |
| Compound 2 | 0.11 | 0.073 |
| Comparison Compound | >10 | >10 |

As shown in the above Table 2, Compound 2 was clarified to inhibit expression of VACM-1 and E-selectin on HUVEC. On the other hand, the VCAM- I expression inhibitory effect of Comparison Compound was not more than 1/90 of that of Compound 2 and E-selectin expression inhibitory effect was not more than 1/130 of the activity of Compound 2.

**Experiment 3:** Effect on thioglycollate (TG) medium-induced infiltration of leukocytes

One (1) ml of TG medium was injected into peritoneal cavity of BALB/c mice on 0 and on day 4, leukocytes that infiltrated into the peritoneal cavity were collected with 3 ml of phosphate-buffered saline containing 0.02% EDTA. The leukocyte concentration (cells/ml) was determined using an automatic blood cell counter. The total number of infiltrated leukocytes (X) was calculated from the concentration of leukocytes (A cells/ml) by the following formula:

$$X=3A$$

assuming that the infiltrated leukocytes were homogeneously floating in the recovered liquids.
Test compounds were suspended in 0.5% hydroxypropylmethylcellulose (Metolose) and administered p.o. (0.1 ml/10 g body weight) on days 0 to 3 after injection of TG medium.
The inventive compound inhibits TO medium-induced infiltration of leukocytes in a dose-dependent manner and suppresses infiltration of leukocytes into inflammatory site by inhibiting cell adhesion also *in vivo.*

**Experiment 4:** Effect on lipopolysaccharide (LPS)-induced adhesion of leukocytes

It has been reported that, by the administration of LPS to mice, an adhesion molecule, Mac-1-dependent leukocyte adhesion is induced, the leukocytes infiltrate into lung, liver and so on, and as a result, the number of peripheral blood leukocytes reduces (Morisaki et al., Clin. Immunol. Immunopathol., 61, 365-375, 1991). The effect on LPS-induced leukocyte adhesion was investigated by taking the reduction of peripheral blood leukocytes as an indicator of leukocyte adhesion.
LPS solution (50 ng/ml in saline, 0.5 ml/mouse) was injected into peritoneal cavity of BALB/c mice. Six hours after the LPS injection, the number of peripheral blood leukocytes was counted with an automatic blood cell counter. Test compounds were suspended in 0.5% hydroxypropylmethylcellulose (Metolose) and administered p.o. (0.1 ml/10 g body weight) from 4 days before LPS injection to the day of LPS injection.
The inventive compounds inhibit the reduction of leukocytes caused by LPS-induced adhesion of leukocytes in a dose-dependent manner.

**Experiment 5**: Effect on trinitrobenzenesulfonic acid (TNBS)-induced colitis in rat

Colitis was induced by intraintestinal injection of TNBS solution (60 mg/ml in 50% EtOH, 500 μl) to SD rats. Seven days later, rats were subjected to autopsy and large intestine (8 cm from anus) was cut out and weighed. Myeloperoxidase (MPO) activity in the intestinal tissue was measured and taken as an indicator of infiltrated leukocyte count. That is, the intestine was homogenized and a cycle of freeze-thawing of the tissue was repeated 3 times. The supernatant obtained by centrifugation was assayed for MPO activity using peroxidase substrate (o-phenylenediamine). MPO unit in the supernatant was calculated with the commercial MPO as a standard. The increase in intestinal weight and increase in MPO activity determined as above were taken as indicators of the degree of colitis. Test compound was suspended in 0.5% hydroxypropylmethylcellulose (Metolose)solution and administered p.o. (0.4 ml/100 mg) or intraintestinally (0.2 ml/rat) for 5 days beginning on the day of TNBS injection. The results of intestinal weight and MPO activity are shown in the following.

(1) Action of Compound 1 by intraintestinal administration

Table 3A

| Treatment (TNBS) | Compound | Dose (mg/site) | Intestinal weight (mg, mean±S.D.) | Significance* |
|---|---|---|---|---|
| nonsensitized | | 0 | 431.3±47.2 | |
| sensitized | | 0 | 2304.2±1375.0 | Control group |
| sensitized | Compound 1 | 0.01 | 501.5±78.3 | P<0.01 |
| sensitized | Compound 1 | 0.1 | 472.0±43.4 | P<0.01 |
| sensitized | Compound 1 | 1 | 460.8±52.3 | P<0.01 |

*: Dunnett's test

Table 3B

| Treatment (TNBS) | Compound | Dose (mg/site) | MPO activity (U/ml, mean±S.D.) | Significance* |
|---|---|---|---|---|
| nonsensitized | | 0 | 0.457±0.079 | |
| sensitized | | 0 | 6.211±5.106 | Control group |
| sensitized | Compound 1 | 0.01 | 0.716±0.158 | P<0.05 |
| sensitized | Compound 1 | 0.1 | 0.654±0.109 | P<0.05 |
| sensitized | Compound 1 | 1 | 0.676±0.172 | P<0.05 |

*: Mann-Whitney's U-test

(2) Action of Compound 2 by intraintestinal administration

Table 4A

| Treatment (TNBS) | Compound | Dose (mg/site) | Intestinal weight (mg, mean ± S.D.) | Significance* |
|---|---|---|---|---|
| nonsensitized | | 0 | 439.9 ± 37.0 | |
| sensitized | | 0 | 704.9 ± 211.1 | Control group |
| sensitized | Compound 2 | 0.01 | 499.6 ± 71.7 | P<0.01 |
| sensitized | Compound 2 | 0.1 | 502.0 ± 51.5 | P<0.01 |
| sensitized | Compound 2 | 1 | 536.0 ± 62.2 | P<0.05 |

*: Dunnett's test

Table 4B

| Treatment (TNBS) | Compound | Dose (mg/site) | MPO activity (U/ml, mean ± S.D.) | Significance* |
|---|---|---|---|---|
| nonsensitized | | 0 | 0.428 ± 0.100 | |
| sensitized | | 0 | 0.796 ± 0.557 | Control group |
| sensitized | Compound 2 | 0.01 | 0.354 ± 0.081 | P<0.01 |

*: Mann-Whitney's U-test

Table 4B (continued)

| Treatment (TNBS) | Compound | Dose (mg/site) | MPO activity (U/ml, mean ± S.D.) | Significance* |
|---|---|---|---|---|
| sensitized | Compound 2 | 0.1 | 0.334 ± 0.069 | P<0.01 |
| sensitized | Compound 2 | 1 | 0.345 ± 0.097 | P<0.01 |

*: Mann-Whitney's U-test

(3) Action of Compound 2 by oral administration

Table 5A

| Treatment (TNBS) | Compound | Dose (mg/kg) | Intestinal weight (mg, mean ± S.D.) | Significance* |
|---|---|---|---|---|
| nonsensitized | | 0 | 402.3 ± 30.6 | |
| sensitized | | 0 | 804.3 ± 381.9 | Control group |
| sensitized | Compound 2 | 0.03 | 699.3 ± 163.7 | none |
| sensitized | Compound 2 | 1 | 468.5 ± 47.6 | P<0.01 |
| sensitized | Compound 2 | 3 | 458.0 ± 34.7 | P<0.01 |

*: Dunnett's test

Table 5B

| Treatment (TNBS) | Compound | Dose (mg/kg) | MPO activity (U/ml, mean ± S.D.) | Significance* |
|---|---|---|---|---|
| nonsensitized | | 0 | 0.434 ± 0.065 | |
| sensitized | | 0 | 1.434 ± 0.651 | Control group |
| sensitized | Compound 2 | 0.3 | 1.568 ± 0.538 | none |
| sensitized | Compound 2 | 1 | 0.769 ± 0.222 | P<0.05 |
| sensitized | Compound 2 | 3 | 0.740 ± 0.142 | P<0.05 |

*: Mann-Whitney's U-test

As shown in the above Tables, Compound 1 and Compound 2 significantly inhibited increase in the intestinal weight and the leukocyte infiltration caused by the onset of colitis in the rats, which suggests the usefulness of the inventive compounds as therapeutic or prophylactic drug for inflammatory bowel diseases (ulcerative colitis and Crohn's disease) also in humans.

**Experiment 6:** Effect on carrageenan-induced colitis in rabbits

A mixed emulsion of 1% λ-degraded carrageenan and Freund's complete adjuvant was subcutaneously injected to rabbits for sensitization. Starting from the $7^{th}$ day (day 0) from sensitization, λ-degraded carrageenan (250-1000 mg/day/rabbit) was forcibly administered orally for 8 weeks to induce colitis. The next day (day 56) of the termination of λ-degraded carrageenan intake, the rabbits were subjected to autopsy and disorder in large intestine was scored using abnormal observation of mucosal epithelium of the tissue sample, level of infiltration of inflammatory cells and the like as indices. The test compounds (10 mg/kg) were consecutively administered from day 0 for 8 weeks. The results are shown in the following.

Table 6

| Compound | mean ± S.E. of score | Significance* |
|---|---|---|
| Control | 10.9 ± 2.0 | |
| Compound 2 | 5.3 ± 0.6 | P<0.05 |

*: Mann-Whitney's U-test

**Experiment 7:** Toxicity test

Compound 2 was repeatedly administered orally to female rats for 10 days. No case of death was observed at the dose of 300 mg/kg. In addition, no abnormality was found in general condition, body weight, feed intake, hematological tests, biochemical tests, urinalysis, organ weight or biopsy.

As is evident from the Experimental Examples as described above, the compound of the present invention has (1) inhibitory effect on leukocyte adhesion to human umbilical vein-derived endothelial cells (HUVEC) by inhibiting the CD11b expression on leukocytes, (2) inhibitory effect on the VCAM-1 and E-selectin expressions on HUVEC, and (3) inhibitory effect on leukocyte infiltration *in vivo*. These effects suggest that the compound of the present invention is useful as a preventive and therapeutic drug for various diseases in which cell adhesion is involved in the onset and progress thereof. In fact, significant suppression of inflammations has been demonstrated in the experiments using colitis model animals. The compound of the formula (1) of the present invention obtained as above and a pharmaceutical agent containing the same are useful as a therapeutic agent, a preventive drug or a recurrence preventive drug for inflammatory bowel diseases caused by cell adhesion, such as ulcerative colitis and Crohn's disease, in mammals inclusive of human, monkey, cow, horse, dog, cat, rabbit, rat and the like. In addition, the inventive compound can be used for the prophylaxis and treatment of venous insufficiency and venous ulcer or maintained remission of the both.

In addition, inasmuch as the low toxicity of the inventive compound has been established by experiments using animals, the compound is useful as a highly safe pharmaceutical product.

This application is based on application No. 243793/1996 filed in Japan.

## Claims

**1.** A thienotriazolodiazepine compound of the formula (1)

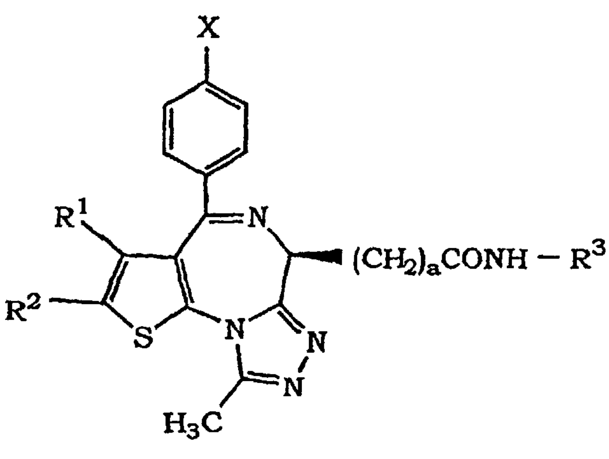

(1)

wherein

- X is a halogen;
- $R^1$ is an alkyl having 1 to 4 carbon atoms;
- $R^2$ is an alkyl having 1 to 4 carbon atoms;
- a is an integer of 1 to 4; and
- $R^3$ is an alkyl having 1 to 4 carbon atoms, a hydroxyalkyl having 1 to 4 carbon atoms, an alkoxy having 1 to 4 carbon atoms, a phenyl, optionally having, as a substltuent, 1 or 2 from halogen, alkyl having 1-4 carbon

atoms, hydroxy, hydroxyalkyl having 1 to 4 carbon atoms, amino, and nitro, or a heteroaryl selected from pyridyl, pyrazinyl, pyrimidinyl, thienyl, and furyl, which heteroaryl optionally having, as a substituent, 1 or 2 from halogen, alkyl having 1 to 4 carbon atoms, hydroxy, amino, nitro, and alkoxy having 1 to 4 carbon atoms,

a pharmaceutically acceptable salt thereof or a hydrate thereof.

**2.** The thienotriazolodiazepine compound of claim 1, wherein, in the formula (1), X is a chlorine and $R^1$ and $R^2$ are both methyl, a pharmaceutically acceptable salt thereof or a hydrate thereof.

**3.** The thienotriazolodiazepine compound of claim 1 or claim 2, wherein, in the formula (1), a is 1, a pharmaceutically acceptable salt thereof or a hydrate thereof.

**4.** The thienotriazolodiazepine compound of claim 1, wherein the compound of the formula (1) is (S)-2-[4-(4-chlorophenyl)-2,3,9-trimethyl-6H-thieno[3,2-f][1,2,4]triazolo[4,3-a][1,4]diazepin-6-yl]-N-(4-hydroxyphenyl)acetamide, a pharmaceutically acceptable salt thereof or a hydrate thereof.

**5.** (S)-2-[4-(4-Chlorophenyl)-2,3,9-trimethyl-6H-thieno[3,2-f][1,2,4]triazolo[4,3-a][1,4]diazepin-6-yl]-N-(4-hydroxyphenyl)acetamide dihydrate.

**6.** A medication comprising a thienotriazolodiazepine compound of the formula (1)

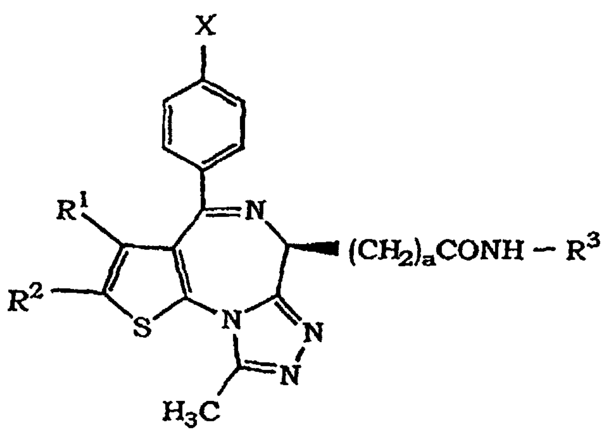

(1)

wherein

X is a halogen;
$R^1$ is an alkyl having 1 to 4 carbon atoms;
$R^2$ is an alkyl having 1 to 4 carbon atoms;
a is an integer of 1 to 4; and
$R^3$ is an alkyl having 1 to 4 carbon atoms, a hydroxyalkyl having 1 to 4 carbon atoms, an alkoxy having 1 to 4 carbon atoms, a phenyl, optionally having, as a substituent, 1 or 2 from halogen, alkyl having 1-4 carbon atoms, hydroxy, hydroxyalkyl having 1 to 4 carbon atoms, amino, and nitro, or a heteroaryl selected from pyridyl, pyrazinyl, pyrimidinyl, thienyl, and furyl, which heteroaryl optionally having, as a substituent, 1 or 2 from halogen, alkyl having 1 to 4 carbon atoms, hydroxy, amino, nitro, and alkoxy having 1 to 4 carbon atoms,

a pharmaceutically acceptable salt thereof or a hydrate thereof.

**7.** The medication of claim 6, comprising a thienotriazolodiazepine compound of the formula (1), wherein X is a chlorine and $R^1$ and $R^2$ are both methyl, a pharmaceutically acceptable salt thereof or a hydrate thereof.

**8.** The medication of claim 6 or claim 7, comprising a thienotriazolodiazepine compound of the formula (1), wherein a is 1, a pharmaceutically acceptable salt thereof or a hydrate thereof.

**9.** The medication of claim 6, wherein the compound of the formula (1) is (S)-2-[4-(4-chlorophenyl)-2,3,9-trimethyl-6H-thieno[3,2-f][1,2,4]triazolo[4,3-a][1,4]diazepin-6-yl]-N-(4-hydroxyphenyl)acetamide.

**10.** The medication of claim 6, which is a therapeutic agent for an inflammatory bowel disease.

**11.** The medication of claim 6, which is a therapeutic agent for an ulcerative colitis.

**12.** The medication of claim 6, which is a therapeutic agent for Crohn's disease.

**13.** A medication comprising (S)-2-[4-(4-chlorophenyl)-2,3,9-trimethyl-6H-thieno[3,2-f][1,2,4]triazolo[4,3-a][1,4]diazepin-6-yl]-N-(4-hydroxyphenyl)acetamide dihydrate.

**14.** The medication of claim 13, which is a therapeutic agent for an inflammatory bowel disease.

**15.** The medication of claim 13, which is a therapeutic agent for an ulcerative colitis.

**16.** The medication of claim 13, which is a therapeutic agent for Crohn's disease.

**Patentansprüche**

**1.** Thienotriazolodiazepin-Verbindung der Formel (1)

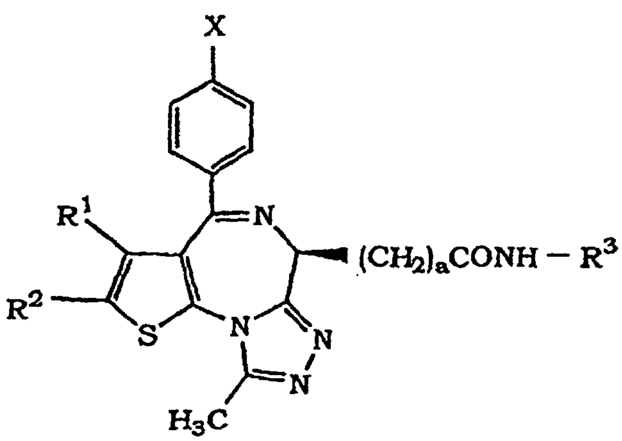

(1)

wobei

X ein Halogen ist;
$R^1$ ein Alkyl mit 1 bis 4 Kohlenstoffatomen ist;
$R^2$ ein Alkyl mit 1 bis 4 Kohlenstoffatomen ist;
a eine ganze Zahl von 1 bis 4 ist; und
$R^3$ ein Alkyl mit 1 bis 4 Kohlenstoffatomen, ein Hydroxyalkyl mit 1 bis 4 Kohlenstoffatomen, ein Alkoxy mit 1 bis 4 Kohlenstoffatomen, ein Phenyl, das gegebenenfalls 1 oder 2 Substituenten aufweist, die aus Halogen, Alkyl mit 1 bis 4 Kohlenstoffatomen, Hydroxy, Hydroxyalkyl mit 1 bis 4 Kohlenstoffatomen, Amino und Nitro ausgewählt sind, oder ein Heteroaryl, das aus Pyridyl, Pyrazinyl, Pyrimidinyl, Thienyl und Furyl ausgewählt ist, ist, wobei das Heteroaryl gegebenenfalls 1 oder 2 Substituenten aufweist, die aus Halogen, Alkyl mit 1 bis 4 Kohlenstoffatomen, Hydroxy, Amino, Nitro und Alkoxy mit 1 bis 4 Kohlenstoffatomen ausgewählt sind;

ein pharmazeutisch annehmbares Salz davon oder ein Hydrat davon.

**2.** Thienotriazolodiazepin-Verbindung gemäß Anspruch 1, wobei X in Formel (1) Chlor ist und $R^1$ und $R^2$ beide Methyl sind, ein pharmazeutisch annehmbares Salz davon oder ein Hydrat davon.

**3.** Thienotrlazolodiazepin-Verbindung gemäß Anspruch 1 oder Anspruch 2, wobei a in Formel (1) gleich 1 ist, ein pharmazeutisch annehmbares Salz. davon oder ein Hydrat davon.

4. Thienotriazoiodiazepin-Verbindung gemäß Anspruch 1, wobei es sich bei der Verbindung der Formel (1) um (S)-2-[4-(4-Chlorphenyl)-2,3,9-trimethyl-6H-thieno[3,2-f][1,2,4]triazolo[4,3-a][1,4]diazepin-6-yl]-N-(4-hydroxyphenyl) acetamid, ein pharmazeutisch annehmbares Salz davon oder ein Hydrat davon handelt.

5. (S)-2-[4-(4-Chlorphenyl)-2,3,9-trimethyl-6H-thieno[3,2-f][1,2,4]triazolo-[4,3-a][1,4]diazepin-6-yl]-N-(4-hydroxyphenyl)acetamid-Dihydrat.

6. Medikament, das eine Thienotriazolodiazepin-Verbindung der Formel (1)

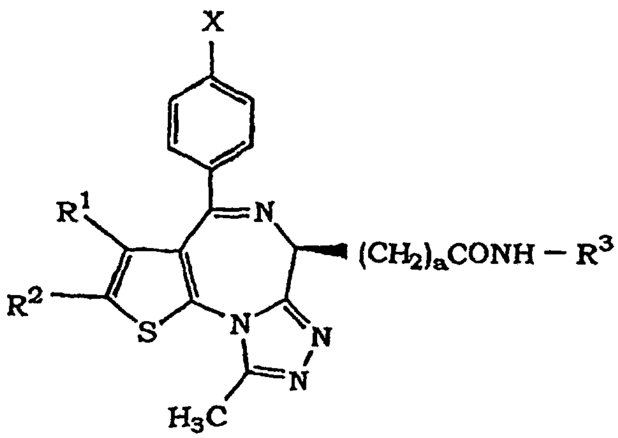

(1)

wobei

X ein Halogen ist;
$R^1$ ein Alkyl mit 1 bis 4 Kohlenstoffatomen ist;
$R^2$ ein Alkyl mit 1 bis 4 Kohlenstoffatomen ist;
a eine ganze Zahl von 1 bis 4 ist; und
$R^3$ ein Alkyl mit 1 bis 4 Kohlenstoffatomen, ein Hydroxyalkyl mit 1 bis 4 Kohlenstoffatomen, ein Alkoxy mit 1 bis 4 Kohlenstoffatomen, ein Phenyl, das gegebenenfalls 1 oder 2 Substituenten aufweist, die aus Halogen, Alkyl mit 1 bis 4 Kohlenstoffatomen, Hydroxy, Hydroxyalkyl mit 1 bis 4 Kohlenstoffatomen, Amino und Nitro ausgewählt sind, oder ein Heteroaryl, das aus Pyridyl, Pyrazinyl, Pyrimidinyl, Thienyl und Furyl ausgewählt ist, ist, wobei das Heteroaryl gegebenenfalls 1 oder 2 Substituenten aufweist, die aus Halogen, Alkyl mit 1 bis 4 Kohlenstoffatomen, Hydroxy, Amino, Nitro und Alkoxy mit 1 bis 4 Kohlenstoffatomen ausgewählt sind;

ein pharmazeutisch annehmbares Salz davon oder ein Hydrat davon umfasst.

7. Medikament gemäß Anspruch 6, das eine Thienotriazolodiazepin-Verbindung der Formel (1), wobei X Chlor ist und $R^1$ und $R^2$ beide Methyl sind, ein pharmazeutisch annehmbares Salz davon oder ein Hydrat davon umfasst.

8. Medikament gemäß Anspruch 6 oder Anspruch 7, das eine Thienotriazolodiazepin-Verbindung der Formel (1), wobei a gleich 1 ist, ein pharmazeutisch annehmbares Salz davon oder ein Hydrat davon umfasst.

9. Medikament gemäß Anspruch 6, wobei es sich bei der Verbindung der Formel (1) um (S)-2-[4-(4-Chlorphenyl)-2,3,9-trimethyl-6H-thieno[3,2-f]-[1,2,4]triazolo[4,3-a][1,4]diazepin-6-yl]-N-(4-hydroxyphenyl)acetamid handelt.

10. Medikament gemäß Anspruch 6, bei dem es sich um ein Therapeutikum für eine chronisch-entzündliche Darmerkrankung handelt.

11. Medikament gemäß Anspruch 6, bei dem es sich um ein Therapeutikum für Colitis ulcerosa handelt.

12. Medikament gemäß Anspruch 6, bei dem es sich um ein Therapeutikum für Morbus Crohn handelt.

13. Medikament, das (S)-2-[4-(4-Chlorphenyl)-2,3,9-trimethyl-6H-thieno[3,2-f][1,2,4]triazolo[4,3-a][1,4]diazepin-6-yl]-N-(4-hydroxyphenyl)acetamid-Dihydrat umfasst.

**14.** Medikament gemäß Anspruch 13, bei dem es sich um ein Therapeutikum für eine chronisch-entzündliche Darmerkrankung handelt.

**15.** Medikament gemäß Anspruch 13, bei dem es sich um ein Therapeutikum für Colitis ulcerosa handelt.

**16.** Medikament gemäß Anspruch 13, bei dem es sich um ein Therapeutikum für Morbus Crohn handelt.

**Revendications**

**1.** Dérivé de thiénotriazolodiazépine de formule (1)

(1)

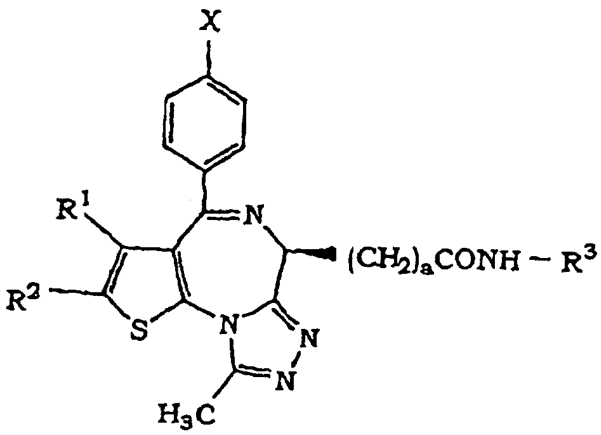

dans laquelle
X représente un atome d'halogène,
$R^1$ représente un groupe alkyle en $C_{1-4}$,
$R^2$ représente un groupe alkyle en $C_{1-4}$,
*a* est un nombre entier entre 1 et 4, et
$R^3$ représente un groupe alkyle en $C_{1-4}$, un groupe hydroxyalkyle en $C_{1-4}$, un groupe alcoxy en $C_{1-4}$, un groupe phényle portant éventuellement 1 ou 2 substituants choisis parmi halogéno, alkyle en $C_{1-4}$, hydroxy, hydroxyalkyle en $C_{1-4}$, amino et nitro, ou un groupe hétéroaryle choisi parmi les groupes pyridyle, pyrazinyle, pyrimidinyle, thiényle et furyle, lequel groupe hétéroaryle portant éventuellement 1 ou 2 substituants choisis parmi halogéno, alkyle en $C_{1-4}$, hydroxy, amino, nitro et alcoxy en $C_{1-4}$,
un sel pharmaceutiquement acceptable ou un hydrate d'un tel composé.

**2.** Dérivé de thiénotriazolodiazépine selon la revendication 1, dans lequel, dans la formule (1), X représente un atome de chlore et $R^1$ et $R^2$ représentent tous les deux un groupe méthyle, un sel pharmaceutiquement acceptable ou un hydrate d'un tel composé.

**3.** Dérivé de thiénotriazolodiazépine selon la revendication 1 ou 2, dans lequel, dans la formule (1), *a* vaut 1, un sel pharmaceutiquement acceptable ou un hydrate d'un tel composé.

**4.** Dérivé de thiénotriazolodiazépine selon la revendication 1, où le composé de formule (1) est le (S)-2-[4-(4-chlorophényl)-2,3,9-triméthyl-6H-thiéno[3,2-f][1,2,4]triazolo[4,3-a][1,4-]diazépin-6-yl]-N-(4-hydroxyphényl)acétamide, un sel pharmaceutiquement acceptable ou un hydrate de celui-ci.

**5.** Dihydrate de (S)-2-[4-(4-chlorophényl)-2,3,9-triméthyl-6H-thiéno[3,2-f][1,2,4]triazolo[4,3-a][1,4]diazépin-6-yl]-N (4-hydroxyphényl)acétamide.

**6.** Médicament comprenant un dérivé de thiénotriazolodiazépine de formule (1)

(1) 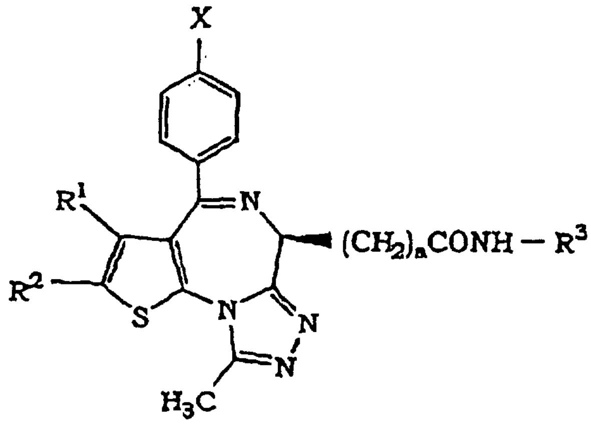

dans laquelle
X représente un atome d'halogène,
$R^1$ représente un groupe alkyle en $C_{1-4}$,
$R^2$ représente un groupe alkyle en $C_{1-4}$,
*a* est un nombre entier entre 1 et 4, et
$R^3$ représente un groupe alkyle en $C_{1-4}$, un groupe hydroxyalkyle en $C_{1-4}$, un groupe alcoxy en $C_{1-4}$, un groupe phényle portant éventuellement 1 ou 2 substituants choisis parmi halogéno, alkyle en $C_{1-4}$, hydroxy, hydroxyalkyle en $C_{1-4}$, amino et nitro, ou un groupe hétéroaryle choisi parmi les groupes pyridyle, pyrazinyle, pyrimidinyle, thiényle et furyle, lequel groupe hétéroaryle portant éventuellement 1 ou 2 substituants choisis parmi halogéno, alkyle en $C_{1-4}$, hydroxy, amino, nitro et alcoxy en $C_{1-4}$,
un sel pharmaceutiquement acceptable ou un hydrate d'un tel composé.

7. Médicament selon la revendication 6, comprenant un dérivé de thiénotriazolodiazépine de formule (1) dans laquelle X représente un atome de chlore et $R^1$ et $R^2$ représentent tous les deux un groupe méthyle, un sel pharmaceutiquement acceptable ou un hydrate d'un tel composé.

8. Médicament selon la revendication 6 ou 7, comprenant un dérivé de thiénotriazolodiazépine de formule (1) dans laquelle *a* vaut 1, un sel pharmaceutiquement acceptable ou un hydrate d'un tel composé.

9. Médicament selon la revendication 6, dans lequel le composé de formule (1) est le (S)-2-[4-(4-chlorophényl)-2,3,9-triméthyl-6H-thiéno[3,2-f][1,2,4]triazolo[4,3-a][1,4]diazépin-6-yl]-N(4-hydroxyphényl)acétamide.

10. Médicament selon la revendication 6 qui est un agent thérapeutique pour traiter les maladies inflammatoires de l'intestin.

11. Médicament selon la revendication 6, qui est un agent thérapeutique pour traiter la rectocolite hémorragique.

12. Médicament selon la revendication 6, qui est un agent thérapeutique pour traiter la maladie de Crohn.

13. Médicament comprenant du dihydrate de (S)-2-[4-(4-chlorophényl)-2,3,9-timéthyl-6H-thiéno[3,2-f][1,2,4]triazolo [4,3-a] [1,4]diazépin-6-yl]-N(4-hydroxyphényl)acétamide.

14. Médicament selon la revendication 13, qui est un agent thérapeutique pour traiter des maladies inflammatoires de l'intestin.

15. Médicament selon la revendication 13, qui est un agent thérapeutique pour traiter la rectocolite hémorragique.

16. Médicament selon la revendication 13, qui est un agent thérapeutique pour traiter la maladie de Crohn.